# EUROPEAN PATENT SPECIFICATION

(11) **EP 2 512 221 B1**
(45) Date of publication and mention of the grant of the patent: **07.11.2018**
(21) Application number: 10838259.9
(22) Date of filing: 16.12.2010
(51) Int. Cl.: C12N 15/82, A01H 5/00, A01H 5/10, A01N 63/02, A01N 65/00

(54) **COMBINED USE OF CRY1DA AND CRY1FA PROTEINS FOR INSECT RESISTANCE MANAGEMENT**
KOMBINIERTE VERWENDUNG VON CRY1DA- UND CRY1FA-PROTEINEN FÜR INSEKTENBEKÄMPFUNGSMANAGEMENT
UTILISATION COMBINÉE DES PROTÉINES CRY1DA ET CRY1FA POUR LA GESTION DE LA RÉSISTANCE DES INSECTES

(30) Priority: 16.12.2009 US 284252 P
(43) Date of publication of application: 24.10.2012
(73) Proprietor: Dow AgroSciences, LLC, Indianapolis, Indiana 46268 (US)
(72) Inventor: MEADE, Thomas, Zionsville IN 46077 (US); NARVA, Kenneth, Zionsville IN 46077 (US); STORER, Nicholas, P., Kensington MD 20895 (US); SHEETS, Joel, J., Zionsville IN 46077 (US); WOOSLEY, Aaron, T., Fishers IN 46038 (US); BURTON, Stephanie, L., Indianapolis IN 46278 (US)
(74) Representative: Zwicker, Jörk
(86) International application number: PCT/US2010/060815
(87) International publication number: WO 2011/075587

(56) References cited:
- EP-A1- 0 400 246
- US-A1- 2003 084 606
- US-A1- 2004 133 942
- US-A1- 2005 155 103
- US-A1- 2005 216 969
- US-A1- 2007 006 340
- US-A1- 2009 165 359
- US-A1- 2010 235 951
- ROUSH R T: "TWO-TOXIN STRATEGIES FOR MANAGEMENT OF INSECTICIDAL TRANSGENIC CROPS: CAN PYRAMIDING SUCCEED WHERE PESTICIDE MIXTURES HAVE NOT?", PHILOSOPHICAL TRANSACTIONS. ROYAL SOCIETY OF LONDON.BIOLOGICAL SCIENCES, ROYAL SOCIETY, LONDON, GB, vol. 353, no. 1376, 29 October 1998 (1998-10-29), pages 1777-1786, XP008032212, ISSN: 0962-8436, DOI: 10.1098/RSTB.1998.0330
- BRAVO A ET AL: "How to cope with insect resistance to Bt toxins?", TRENDS IN BIOTECHNOLOGY, ELSEVIER PUBLICATIONS, CAMBRIDGE, GB, vol. 26, no. 10, 2 October 2008 (2008-10-02), pages 573-579, XP025406825, ISSN: 0167-7799, DOI: 10.1016/J.TIBTECH.2008.06.005 [retrieved on 2008-08-14]
- GUTIERREZ ET AL.: 'Physiologically based demographics of Bt cotton-pest interactions I. Pink bollworm resistance, refuge and risk' ECOLOGICAL MODELLING vol. 191, no. 3-4, 05 February 2006, pages 346 - 359, XP005239868

<110> Dow AGROSCIENCES LLC
<120> COMBINED USE OF Cry1Fa AND Cry1Da FOR MANAGEMENT OF RESISTANT INSECTS
<130> DAS-P0161-US
<160> 2
<170> PatentIn version 3.5
<210> 1
   <211> 605
   <212> PRT
   <213> Artificial Sequence
<220>
   <223> Cry1Fa
<400> 1
   Met Glu Asn Asn Ile Gln Asn Gln Cys Val Pro Tyr Asn Cys Leu Asn
   1               5                   10                  15
   Asn Pro Glu Val Glu Ile Leu Asn Glu Glu Arg Ser Thr Gly Arg Leu
               20                  25                  30
   Pro Leu Asp Ile Ser Leu Ser Leu Thr Arg Phe Leu Leu Ser Glu Phe
           35                  40                  45
   Val Pro Gly Val Gly Val Ala Phe Gly Leu Phe Asp Leu Ile Trp Gly
       50                  55                  60
   Phe Ile Thr Pro Ser Asp Trp Ser Leu Phe Leu Leu Gln Ile Glu Gln
   65                  70                  75                  80
   Leu Ile Glu Gln Arg Ile Glu Thr Leu Glu Arg Asn Arg Ala Ile Thr
                   85                  90                  95
   Thr Leu Arg Gly Leu Ala Asp Ser Tyr Glu Ile Tyr Ile Glu Ala Leu
               100                 105                 110
   Arg Glu Trp Glu Ala Asn Pro Asn Asn Ala Gln Leu Arg Glu Asp Val
           115                 120                 125
   Arg Ile Arg Phe Ala Asn Thr Asp Asp Ala Leu Ile Thr Ala Ile Asn
       130                 135                 140
   Asn Phe Thr Leu Thr Ser Phe Glu Ile Pro Leu Leu Ser Val Tyr Val
   145                 150                 155                 160
   Gln Ala Ala Asn Leu His Leu Ser Leu Leu Arg Asp Ala Val Ser Phe
                   165                 170                 175
   Gly Gln Gly Trp Gly Leu Asp Ile Ala Thr Val Asn Asn His Tyr Asn
               180                 185                 190
   Arg Leu Ile Asn Leu Ile His Arg Tyr Thr Lys His Cys Leu Asp Thr
           195                 200                 205
   Tyr Asn Gln Gly Leu Glu Asn Leu Arg Gly Thr Asn Thr Arg Gln Trp
       210                 215                 220
   Ala Arg Phe Asn Gln Phe Arg Arg Asp Leu Thr Leu Thr Val Leu Asp
   225                 230                 235                 240
   Ile Val Ala Leu Phe Pro Asn Tyr Asp Val Arg Thr Tyr Pro Ile Gln
                   245                 250                 255
   Thr Ser Ser Gln Leu Thr Arg Glu Ile Tyr Thr Ser Ser Val Ile Glu
               260                 265                 270
   Asp Ser Pro Val Ser Ala Asn Ile Pro Asn Gly Phe Asn Arg Ala Glu
           275                 280                 285
   Phe Gly Val Arg Pro Pro His Leu Met Asp Phe Met Asn Ser Leu Phe
       290                 295                 300
   Val Thr Ala Glu Thr Val Arg Ser Gln Thr Val Trp Gly Gly His Leu
   305                 310                 315                 320
   Val Ser Ser Arg Asn Thr Ala Gly Asn Arg Ile Asn Phe Pro Ser Tyr
                   325                 330                 335
   Gly Val Phe Asn Pro Gly Gly Ala Ile Trp Ile Ala Asp Glu Asp Pro
               340                 345                 350
   Arg Pro Phe Tyr Arg Thr Leu Ser Asp Pro Val Phe Val Arg Gly Gly
           355                 360                 365
   Phe Gly Asn Pro His Tyr Val Leu Gly Leu Arg Gly Val Ala Phe Gln
       370                 375                 380
   Gln Thr Gly Thr Asn His Thr Arg Thr Phe Arg Asn Ser Gly Thr Ile
   385                 390                 395                 400
   Asp Ser Leu Asp Glu Ile Pro Pro Gln Asp Asn Ser Gly Ala Pro Trp
                   405                 410                 415
   Asn Asp Tyr Ser His Val Leu Asn His Val Thr Phe Val Arg Trp Pro
               420                 425                 430
   Gly Glu Ile Ser Gly Ser Asp Ser Trp Arg Ala Pro Met Phe Ser Trp
               435                 440                 445
   Thr His Arg Ser Ala Thr Pro Thr Asn Thr Ile Asp Pro Glu Arg Ile
           450                 455                 460
   Thr Gln Ile Pro Leu Val Lys Ala His Thr Leu Gln Ser Gly Thr Thr
   465                 470                 475                 480
   Val Val Arg Gly Pro Gly Phe Thr Gly Gly Asp Ile Leu Arg Arg Thr
                   485                 490                 495
   Ser Gly Gly Pro Phe Ala Tyr Thr Ile Val Asn Ile Asn Gly Gln Leu
               500                 505                 510
   Pro Gln Arg Tyr Arg Ala Arg Ile Arg Tyr Ala Ser Thr Thr Asn Leu
           515                 520                 525
   Arg Ile Tyr Val Thr Val Ala Gly Glu Arg Ile Phe Ala Gly Gln Phe
       530                 535                 540
   Asn Lys Thr Met Asp Thr Gly Asp Pro Leu Thr Phe Gln Ser Phe Ser
   545                 550                 555                 560
   Tyr Ala Thr Ile Asn Thr Ala Phe Thr Phe Pro Met Ser Gln Ser Ser
                   565                 570                 575
   Phe Thr Val Gly Ala Asp Thr Phe Ser Ser Gly Asn Glu Val Tyr Ile
               580                 585                 590
   Asp Arg Phe Glu Leu Ile Pro Val Thr Ala Thr Leu Glu
           595                 600                 605
<210> 2
   <211> 594
   <212> PRT
   <213> Artificial Sequence
<220>
   <223> Cry1Da
<400> 2
   Met Glu Ile Asn Asn Gln Asn Gln Cys Val Pro Tyr Asn Cys Leu Ser
   1               5                   10                  15
   Asn Pro Lys Glu Ile Ile Leu Gly Glu Glu Arg Leu Glu Thr Gly Asn
               20                  25                  30
   Thr Val Ala Asp Ile Ser Leu Gly Leu Ile Asn Phe Leu Tyr Ser Asn
           35                  40                  45
   Phe Val Pro Gly Gly Gly Phe Ile Val Gly Leu Leu Glu Leu Ile Trp
       50                  55                  60
   Gly Phe Ile Gly Pro Ser Gln Trp Asp Ile Phe Leu Ala Gln Ile Glu
   65                  70                  75                  80
   Gln Leu Ile Ser Gln Arg Ile Glu Glu Phe Ala Arg Asn Gln Ala Ile
                   85                  90                  95
   Ser Arg Leu Glu Gly Leu Ser Asn Leu Tyr Lys Val Tyr Val Arg Ala
               100                 105                 110
   Phe Ser Asp Trp Glu Lys Asp Pro Thr Asn Pro Ala Leu Arg Glu Glu
           115                 120                 125
   Met Arg Ile Gln Phe Asn Asp Met Asn Ser Ala Leu Ile Thr Ala Ile
       130                 135                 140
   Pro Leu Phe Arg Val Gln Asn Tyr Glu Val Ala Leu Leu Ser Val Tyr
   145                 150                 155                 160
   Val Gln Ala Ala Asn Leu His Leu Ser Ile Leu Arg Asp Val Ser Val
                   165                 170                 175
   Phe Gly Glu Arg Trp Gly Tyr Asp Thr Ala Thr Ile Asn Asn Arg Tyr
               180                 185                 190
   Ser Asp Leu Thr Ser Leu Ile His Val Tyr Thr Asn His Cys Val Asp
           195                 200                 205
   Thr Tyr Asn Gln Gly Leu Arg Arg Leu Glu Gly Arg Phe Leu Ser Asp
       210                 215                 220
   Trp Ile Val Tyr Asn Arg Phe Arg Arg Gln Leu Thr Ile Ser Val Leu
   225                 230                 235                 240
   Asp Ile Val Ala Phe Phe Pro Asn Tyr Asp Ile Arg Thr Tyr Pro Ile
                   245                 250                 255
   Gln Thr Ala Thr Gln Leu Thr Arg Glu Val Tyr Leu Asp Leu Pro Phe
               260                 265                 270
   Ile Asn Glu Asn Leu Ser Pro Ala Ala Ser Tyr Pro Thr Phe Ser Ala
           275                 280                 285
   Ala Glu Ser Ala Ile Ile Arg Ser Pro His Leu Val Asp Phe Leu Asn
       290                 295                 300
   Ser Phe Thr Ile Tyr Thr Asp Ser Leu Ala Arg Tyr Ala Tyr Trp Gly
   305                 310                 315                 320
   Gly His Leu Val Asn Ser Phe Arg Thr Gly Thr Thr Thr Asn Leu Ile
                   325                 330                 335
   Arg Ser Pro Leu Tyr Gly Arg Glu Gly Asn Thr Glu Arg Pro Val Thr
               340                 345                 350
   Ile Thr Ala Ser Pro Ser Val Pro Ile Phe Arg Thr Leu Ser Tyr Ile
           355                 360                 365
   Thr Gly Leu Asp Asn Ser Asn Pro Val Ala Gly Ile Glu Gly Val Glu
       370                 375                 380
   Phe Gln Asn Thr Ile Ser Arg Ser Ile Tyr Arg Lys Ser Gly Pro Ile
   385                 390                 395                 400
   Asp Ser Phe Ser Glu Leu Pro Pro Gln Asp Ala Ser Val Ser Pro Ala
                   405                 410                 415
   Ile Gly Tyr Ser His Arg Leu Cys His Ala Thr Phe Leu Glu Arg Ile
               420                 425                 430
   Ser Gly Pro Arg Ile Ala Gly Thr Val Phe Ser Trp Thr His Arg Ser
           435                 440                 445
   Ala Ser Pro Thr Asn Glu Val Ser Pro Ser Arg Ile Thr Gln Ile Pro
       450                 455                 460
   Trp Val Lys Ala His Thr Leu Ala Ser Gly Ala Ser Val Ile Lys Gly
   465                 470                 475                 480
   Pro Gly Phe Thr Gly Gly Asp Ile Leu Thr Arg Asn Ser Met Gly Glu
                   485                 490                 495
   Leu Gly Thr Leu Arg Val Thr Phe Thr Gly Arg Leu Pro Gln Ser Tyr
               500                 505                 510
   Tyr Ile Arg Phe Arg Tyr Ala Ser Val Ala Asn Arg Ser Gly Thr Phe
           515                 520                 525
   Arg Tyr Ser Gln Pro Pro Ser Tyr Gly Ile Ser Phe Pro Lys Thr Met
       530                 535                 540
   Asp Ala Gly Glu Pro Leu Thr Ser Arg Ser Phe Ala His Thr Thr Leu
   545                 550                 555                 560
   Phe Thr Pro Ile Thr Phe Ser Arg Ala Gln Glu Glu Phe Asp Leu Tyr
   565 570 575
   Ile Gln Ser Gly Val Tyr Ile Asp Arg Ile Glu Phe Ile Pro Val Thr
   580 585 590
   Ala Thr

## Description

### Background of the Invention

Humans grow corn for food and energy applications. Humans also grow many other crops, including soybeans and cotton. Insects eat and damage plants and thereby undermine these human efforts. Billions of dollars are spent each year to control insect pests and additional billions are lost to the damage they inflict. Synthetic organic chemical insecticides have been the primary tools used to control insect pests but biological insecticides, such as the insecticidal proteins derived from *Bacillus thuringiensis* (*Bt*), have played an important role in some areas. The ability to produce insect-resistant plants through transformation with *Bt* insecticidal protein genes has revolutionized modern agriculture and heightened the importance and value of insecticidal proteins and their genes.

Several *Bt* proteins have been used to create the insect-resistant transgenic plants that have been successfully registered and commercialized to date. These include Cry1Ab, Cry1Ac, Cry1F and Cry3Bb in corn, Cry1Ac and Cry2Ab in cotton, and Cry3A in potato.

The commercial products expressing these proteins express a single protein except in cases where the combined insecticidal spectrum of 2 proteins is desired (*e.g.,* Cry1Ab and Cry3Bb in corn combined to provide resistance to lepidopteran pests and rootworm, respectively) or where the independent action of the proteins makes them useful as a tool for delaying the development of resistance in susceptible insect populations (*e.g.,* Cry1Ac and Cry2Ab in cotton combined to provide resistance management for tobacco budworm).

That is, some of the qualities of insect-resistant transgenic plants that have led to rapid and widespread adoption of this technology also give rise to the concern that pest populations will develop resistance to the insecticidal proteins produced by these plants. Several strategies have been suggested for preserving the utility of *Bt*-based insect resistance traits which include deploying proteins at a high dose in combination with a refuge, and alternation with, or co-deployment of, different toxins (McGaughey et al. (1998), "B.t. Resistance Management," Nature Biotechnol. 16:144-146).

The proteins selected for use in an IRM stack need to exert their insecticidal effect independently so that resistance developed to one protein does not confer resistance to the second protein (*i.e.,* there is not cross resistance to the proteins). If, for example, a pest population selected for resistance to "Protein A" is sensitive to "Protein B", one would conclude that there is not cross resistance and that a combination of Protein A and Protein B would be effective in delaying resistance to Protein A alone.

In the absence of resistant insect populations, assessments can be made based on other characteristics presumed to be related to mechanism of action and cross-resistance potential. The utility of receptor-mediated binding in identifying insecticidal proteins likely to not exhibit cross resistance has been suggested (van Mellaert *et al.* 1999). The key predictor of lack of cross resistance inherent in this approach is that the insecticidal proteins do not compete for receptors in a sensitive insect species.

In the event that two *Bt* toxins compete for the same receptor, then if that receptor mutates in that insect so that one of the toxins no longer binds to that receptor and thus is no longer insecticidal against the insect, it might be the case that the insect will also be resistant to the second toxin (which competitively bound to the same receptor). That is, the insect is said to be cross-resistant to both *Bt* toxins. However, if two toxins bind to two different receptors, this could be an indication that the insect would not be simultaneously resistant to those two toxins.

Cry1Fa is useful in controlling many lepidopteran pests species including the European corn borer (ECB; *Ostrinia nubilalis* (Hübner)) and the fall armyworm (FAW; *Spodoptera frugiperda*), and is active against the sugarcane borer (SCB; *Diatraea saccharalis).* The Cry1Fa protein, as produced in corn plants containing event TC1507, is responsible for an industry-leading insect resistance trait for FAW control. Cry1Fa is further deployed in the Herculex®, SmartStax™, and WideStrike™ products.

The ability to conduct (competitive or homologous) receptor binding studies using CrylFa protein is limited because the most common technique available for labeling proteins for detection in receptor binding assays inactivates the insecticidal activity of the Cry1Fa protein.

Additional *Cry* toxins are listed at the website of the official *B.t.* nomenclature committee (Crickmore *et al.;* lifesci.sussex.ac.uk/home/Neil_Crickmore/Bt/). *See* Appendix A, attached. There are currently nearly 60 main groups of "Cry" toxins (Cry1-Cry59), with additional Cyt toxins and VIP toxins and the like. Many of each numeric group have capital-letter subgroups, and the capital letter subgroups have lower-cased letter sub-subgroups. (Cry1 has A-L, and Cry1A has a-i, for example).

### Brief Summary of the Invention

The subject invention relates in part to the surprising discovery that a fall armyworm (*Spodoptera frugiperda;* FAW) population selected for resistance to the insecticidal activity of the Cry1Fa protein is not resistant to the insecticidal activity of the Cry1Da protein. As one skilled in the art will recognize with the benefit of this disclosure, plants expressing these two insecticidal proteins, or insecticidal portions thereof, will be useful in delaying or preventing the development of resistance to either of these insecticidal proteins alone.

The subject invention is also supported by the discovery that Cry1Fa and Cry1Da do not compete with each other for binding gut receptors from FAW.

The subject invention also relates in part to triple stacks or "pyramids" of three (or more) toxins, with Cry1Fa and Cry1Da toxins being the base pair. One preferred pyramid provides at least two proteins providing non-cross-resistant activity against two pests - the FAW and the ECB (European corn borer; *Ostrinia nubilalis*): Cry1Fa plus Cry1Da plus one or more anti-ECB toxins such as Cry1Ab. In some preferred pyramid embodiments, the selected toxins have three separate modes of action against FAW. These preferred "three modes of action" pyramid combinations are Cry1Fa plus Cry1D plus another toxin/gene selected from the group consisting of Vip3Ab, Cry1C, Cry1Be, and Cry1E. Plants (and acreage planted with such plants) that produce these three toxins are included within the scope of the subject invention. Additional toxins/genes can also be added, but these particular triple stacks would, according to the subject invention, advantageously and surprisingly provide three modes of action against FAW. This can help to reduce or eliminate the requirement for refuge acreage. The subject invention also relates generally to the use of three insecticidal proteins (*Cry* proteins in some preferred embodiments) that do not compete with each other against a single target pest.

Thus, Cry1Da could be used as in the 3 gene combination for corn and other plants (cotton and soybeans, for example). A cry1Da gene could be combined into, for example, a Cry1Fa product such as Herculex®, SmartStax™, and WidesStrike™. Accordingly, use of Cry1Da could be significant in reducing the selection pressure on other commercialized proteins.

### BRIEF DESCRIPTION OF THE FIGURES

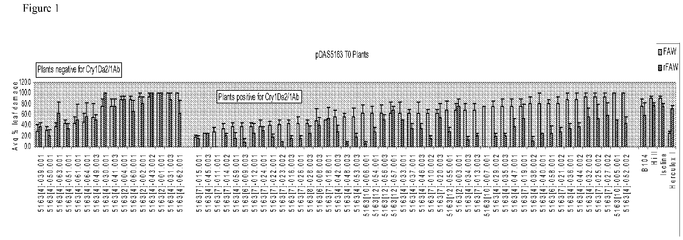

**Figure 1****:** Damage (mean % leaf damage + SEM) to corn leaf segments infested with FAW (blue bars) or rFAW (purple bars). All treatments preceded by the numbers "5163" are leaf segments from plants transformed with a construct containing Cry1Da. Plants in which no Cry1Da expression was detected are grouped on the far left of the graph. Plants in which Cry1Da expression was detected are grouped in the center of the graph. Non-transgenic (i.e., negative) controls are on the far right of the graph and are labeled "B104", "Hill", and "Isoline". A commercial inbred containing Cry1Fa is the first treatment on right (labeled "Herculex I") and is the same genetic background as the non-transgenic control labeled "Isoline".

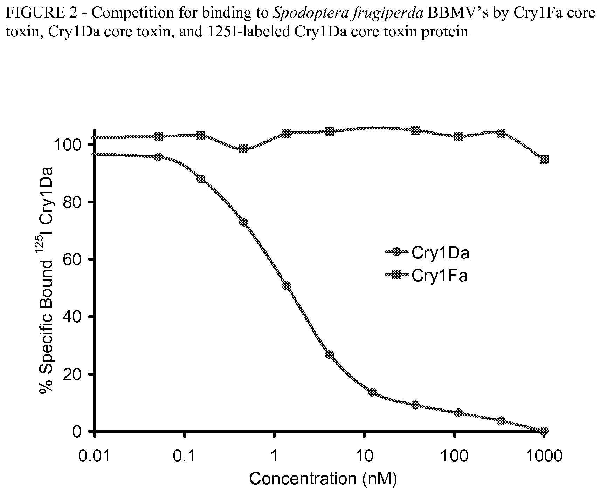

**Figure 2****:** Competition for binding to *Spodoptera frugiperda* BBMV's by Cry1Fa core toxin, Cry1Da core toxin, and 125I-labeled CrylDa core toxin protein

### DETAILED DESCRIPTION OF THE INVENTION

As reported herein, Cry1Da toxin produced in transgenic corn (and other plants; cotton and soybeans, for example) is very effective in controlling fall armyworm (FAW; *Spodoptera frugiperda)* that have developed resistance to Cry1Fa activity. Thus, the subject invention relates in part to the surprising discovery that fall armyworm resistant to Cry1Fa are susceptible (*i.e.,* are not cross-resistant) to Cry1Da.

The subject invention also relates in part to the surprising discovery that Cry1Da toxin is effective at protecting plants (such as maize plants) from damage by Cry1Fa-resistant fall armyworm. For a discussion of this pest, *see e.g.* Tabashnik, PNAS (2008), vol. 105 no. 49, 19029-19030.

The subject invention includes the use of Cry1Da toxin to protect corn and other economically important plant species from damage and yield loss caused by fall armyworm feeding or to fall armyworm populations that have developed resistance to Cry1Fa.

The subject invention thus teaches an IRM stack to prevent or mitigate the development of resistance by fall armyworm to Cry1Fa and/or Cry1Da.

The present invention provides the use of a composition for controlling lepidopteran pests comprising cells that produce a Cry1Fa core toxin-containing protein and a Cry1Da core toxin-containing protein.

The invention further comprises a transgenic plant or plant cell transformed to produce both a Cry1Fa core toxin-containing protein and a Cry1Da core toxin-containing protein, wherein the cry1Fa polynucleotide and the cry1Da polynucleotide are preferably in a genetic construct under control of (operably linked to / comprising) a *non-Bacillus-thuringiensis* promoter(s). The polynucleotides can comprise codon usage for enhanced expression in a plant.

It is additionally intended that the invention provides a method of controlling lepidopteran pests comprising contacting said pests or the environment of said pests with an effective amount of a composition that contains a Cry1Fa core toxin-containing protein and further contains a Cry1Da core toxin-containing protein.

The disclosure comprises a maize plant comprising a plant-expressible gene encoding a Cry1Da core toxin-containing protein and a plant-expressible gene encoding a Cry1Fa core toxin-containing protein, and seed of such a plant.

A further disclosure comprises a maize plant wherein a plant-expressible gene encoding a Cry1Da core toxin-containing protein and a plant-expressible gene encoding a Cry1Fa core toxin-containing protein have been introgressed into said maize plant, and seed of such a plant.

Insect receptors. As described in the Examples, competitive receptor binding studies using radiolabeled Cry1Da core toxin protein show that the Cry1Fa core toxin protein does not compete for the high affinity binding site present in FAW insect tissues to which Cry1Da binds. These results indicate that the combination of Cry1Fa and Cry1Da proteins is an effective means to mitigate the development of resistance in FAW populations to Cry1Fa (and likewise, the development of resistance to Cry1Da), and would likely increase the level of resistance to this pest in corn plants expressing both proteins.

Thus, based in part on the data described above and elsewhere herein, it is thought that co-production (stacking) of the Cry1Da and Cry1Fa proteins can be used to produce a high dose IRM stack for FAW. Other proteins can be added to this combination to expand insect-control spectrum. For example in corn, the addition of Cry1Ab would create an IRM pyramid for control of European corn borer.

Another deployment option would be to use Cry1Fa and Cry1Da proteins in combination with another, third toxin/gene, and to use this triple stack to mitigate the development of resistance in FAW to any of these toxins. Thus, another deployment option of the subject invention would be to use one, two, or three (or more) of these proteins in crop-growing regions where FAW can develop resistant populations. Accordingly, the subject invention also relates in part to triple stacks or "pyramids" of three (or more) toxins, with Cry1Fa and Cry1Da toxins being the base pair. One preferred pyramid provides at least two proteins providing non-cross-resistant activity against two pests - the FAW and the ECB (European corn borer; *Ostrinia nubilalis*): Cry1Fa plus Cry1Da plus one or more ECB toxins such as Cry1Ab (see US 2008 0311096), as Cry1F is active against both insects. Other ECB toxins include Cry1Be (see USSN 61/284,290; filed December 16, 2009), Cry1I (see USSN 61/284,278; filed December 16, 2009), Cry2Aa (see USSN 61/284,278; filed December 16, 2009) and DIG-3 (see US 2010 00269223). In some preferred pyramid embodiments, the selected toxins have three separate modes of action against FAW. These preferred "three modes of action" pyramid combinations are Cry1Fa plus Cry1D plus another toxin/gene selected from the group consisting of Vip3Ab, Cry1C (see USSN 61/284,281; filed December 16, 2009), Cry1Be, and Cry1E (see USSN 61/284,278; filed December 16, 2009). Plants (and acreage planted with such plants) that produce these three toxins are included within the scope of the subject invention. Additional toxins/genes can also be added, but these particular triple stacks would, according to the subject invention, advantageously and surprisingly provide three modes of action against FAW. This can help to reduce or eliminate the requirement for refuge acreage. A field thus planted of over 10 acres is thus included within the subject invention.

Thus, Cry1Da could be used as in the 3 gene combination for corn that currently in the Development I of the new Trait Development process. Cry1Fa is in the Herculex®, SmartStax™, and WidesStrike™ products. Accordingly, use of Cry1Da could be significant in reducing the selection pressure on other commercialized proteins.

Other Vip3 toxins, for example, are listed in the attached Appendix A. Those GENBANK numbers can also be used to obtain the sequences for any of the genes and proteins disclosed or mentioned herein.

U.S. Patent No. 5,188,960 and U.S. Patent No. 5,827,514 describe Cry1Fa core toxin containing proteins suitable for use in carrying out the present invention. U.S. Patent No. 6,218,188 describes plant-optimized DNA sequences encoding Cry1Fa core toxin-containing proteins that are suitable for use in the present invention.

Combinations of the toxins described in the subject invention can be used to control lepidopteran pests. Adult lepidopterans, for example, butterflies and moths, primarily feed on flower nectar and are a significant effector of pollination. Nearly all lepidopteran larvae, *i.e.,* caterpillars, feed on plants, and many are serious pests. Caterpillars feed on or inside foliage or on the roots or stem of a plant, depriving the plant of nutrients and often destroying the plant's physical support structure. Additionally, caterpillars feed on fruit, fabrics, and stored grains and flours, ruining these products for sale or severely diminishing their value. As used herein, reference to lepidopteran pests refers to various life stages of the pest, including larval stages.

Some chimeric toxins comprise a full N-terminal core toxin portion of a *Bt* toxin and, at some point past the end of the core toxin portion, the protein has a transition to a heterologous protoxin sequence. The N-terminal, insecticidally active, toxin portion of a *Bt* toxin is referred to as the "core" toxin. The transition from the core toxin segment to the heterologous protoxin segment can occur at approximately the toxin/protoxin junction or, in the alternative, a portion of the native protoxin (extending past the core toxin portion) can be retained, with the transition to the heterologous protoxin portion occurring downstream.

As an example, one chimeric toxin is a full core toxin portion of Cry1Fa (amino acids 1 to 601) and a heterologous protoxin (amino acids 602 to the C-terminus). The portion of a chimeric toxin comprising the protoxin is derived from a Cry1Ab protein toxin. As a second Example, a second chimeric toxin has the full core toxin portion of Cry1Da (amino acids 1 to 619) and a heterologous protoxin (amino acids 620 to the C-terminus). The portion of a chimeric toxin comprising the protoxin is derived from a Cry1Ab protein toxin.

A person skilled in this art will appreciate that *Bt* toxins, even within a certain class such as Cry1F, will vary to some extent in length and the precise location of the transition from core toxin portion to protoxin portion. Typically, the Cry1Da and Cry1Fa toxins are about 1150 to about 1200 amino acids in length. The transition from core toxin portion to protoxin portion will typically occur at between about 50% to about 60% of the full length toxin. The chimeric toxin of the subject invention will include the full expanse of this N-terminal core toxin portion. Thus, the chimeric toxin will comprise at least about 50% of the full length of the Cry1Fa *Bt* toxin protein or at least about 50% of the full length of the Cry1Da *Bt* toxin protein. This will typically be at least about 590 amino acids. With regard to the protoxin portion, the full expanse of the Cry1Ab protoxin portion extends from the end of the core toxin portion to the C-terminus of the molecule.

Genes and toxins. The genes and toxins include not only the full length sequences disclosed but also fragments of these sequences, variants, mutants, and fusion proteins which retain the characteristic pesticidal activity of the toxins specifically exemplified herein. As used herein, the terms "variants" or "variations" of genes refer to nucleotide sequences which encode the same toxins or which encode equivalent toxins having pesticidal activity. As used herein, the term "equivalent toxins" refers to toxins having the same or essentially the same biological activity against the target pests as the claimed toxins.

As used herein, the boundaries represent approximately 95% (Cry1Fa's and 1Da's), 78% (Cry1F's and Cry1D's), and 45% (Cry1's) sequence identity, per "Revision of the Nomenclature for the Bacillus thuringiensis Pesticidal Crystal Proteins," N. Crickmore, D.R. Zeigler, J. Feitelson, E. Schnepf, J. Van Rie, D. Lereclus, J. Baum, and D.H. Dean. Microbiology and Molecular Biology Reviews (1998) Vol 62: 807-813. These cut offs can also be applied to the core toxins only (for Cry1F and Cry1D toxins).

It should be apparent to a person skilled in this art that genes encoding active toxins can be identified and obtained through several means. The specific genes or gene portions exemplified herein may be obtained from the isolates deposited at a culture depository. These genes, or portions or variants thereof, may also be constructed synthetically, for example, by use of a gene synthesizer. Variations of genes may be readily constructed using standard techniques for making point mutations. Also, fragments of these genes can be made using commercially available exonucleases or endonucleases according to standard procedures. For example, enzymes such as Bal31 or site-directed mutagenesis can be used to systematically cut off nucleotides from the ends of these genes. Genes that encode active fragments may also be obtained using a variety of restriction enzymes. Proteases may be used to directly obtain active fragments of these protein toxins.

Fragments and equivalents which retain the pesticidal activity of the exemplified toxins are disclosed herein. Also, because of the redundancy of the genetic code, a variety of different DNA sequences can encode the amino acid sequences disclosed herein. It is well within the skill of a person trained in the art to create these alternative DNA sequences encoding the same, or essentially the same, toxins. These variant DNA sequences are within the scope of the subject invention. As used herein, reference to "essentially the same" sequence refers to sequences which have amino acid substitutions, deletions, additions, or insertions which do not materially affect pesticidal activity. Fragments of genes encoding proteins that retain pesticidal activity are also included in this definition.

A further method for identifying the genes encoding the toxins and gene portions useful according to the subject invention is through the use of oligonucleotide probes. These probes are detectable nucleotide sequences. These sequences may be detectable by virtue of an appropriate label or may be made inherently fluorescent as described in International Application No. WO93/16094. As is well known in the art, if the probe molecule and nucleic acid sample hybridize by forming a strong bond between the two molecules, it can be reasonably assumed that the probe and sample have substantial homology. Preferably, hybridization is conducted under stringent conditions by techniques well-known in the art, as described, for example, in Keller, G. H., M. M. Manak (1987) DNA Probes, Stockton Press, New York, N.Y., pp. 169-170. Some examples of salt concentrations and temperature combinations are as follows (in order of increasing stringency): 2X SSPE or SSC at room temperature; 1X SSPE or SSC at 42° C; 0.1X SSPE or SSC at 42° C; 0.1X SSPE or SSC at 65° C. Detection of the probe provides a means for determining in a known manner whether hybridization has occurred. Such a probe analysis provides a rapid method for identifying toxin-encoding genes of the subject invention. The nucleotide segments which are used as probes according to the invention can be synthesized using a DNA synthesizer and standard procedures. These nucleotide sequences can also be used as PCR primers to amplify genes of the subject invention.

Variant toxins. Certain toxins have been specifically exemplified herein. Equivalent toxins will have amino acid homology with an exemplified toxin. This amino acid homology will typically be greater than 75%, preferably be greater than 90%, and most preferably be greater than 95%. The amino acid homology will be highest in critical regions of the toxin which account for biological activity or are involved in the determination of three-dimensional configuration which ultimately is responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject invention so long as the substitution does not materially alter the biological activity of the compound. Below is a listing of examples of amino acids belonging to each class.

| **Class of Amino Acid** | **Examples of Amino Acids** |
|---|---|
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |
| Basic | Lys, Arg, His |

In some instances, non-conservative substitutions can also be made. The critical factor is that these substitutions must not significantly detract from the biological activity of the toxin.

Recombinant hosts. The genes encoding the toxins of the subject invention can be introduced into a wide variety of microbial or plant hosts. Expression of the toxin gene results, directly or indirectly, in the intracellular production and maintenance of the pesticide. Conjugal transfer and recombinant transfer can be used to create a *Bt* strain that expresses both toxins of the subject invention. Other host organisms may also be transformed with one or both of the toxin genes then used to accomplish the synergistic effect. With suitable microbial hosts, *e.g., Pseudomonas,* the microbes can be applied to the situs of the pest, where they will proliferate and be ingested. The result is control of the pest. Alternatively, the microbe hosting the toxin gene can be treated under conditions that prolong the activity of the toxin and stabilize the cell. The treated cell, which retains the toxic activity, then can be applied to the environment of the target pest.

Where the *Bt* toxin gene is introduced via a suitable vector into a microbial host, and said host is applied to the environment in a living state, it is essential that certain host microbes be used. Microorganism hosts are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, *e.g.,* genera *Pseudomonas, Erwinia, Serratia, Klebsiella, Xanthomonas, Streptomyces, Rhizobium, Rhodopseudomonas, Methylophilius, Agrobactenum, Acetobacter, Lactobacillus, Arthrobacter, Azotobacter, Leuconostoc,* and *Alcaligenes;* fungi, particularly yeast, *e.g.,* genera *Saccharomyces, Cryptococcus, Kluyveromyces, Sporobolomyces, Rhodotorula,* and *Aureobasidium.* Of particular interest are such phytosphere bacterial species as *Pseudomonas syringae, Pseudomonas fluorescens, Serratia marcescens, Acetobacter xylinum, Agrobactenium tumefaciens, Rhodopseudomonas spheroides, Xanthomonas campestris, Rhizobium melioti, Alcaligenes entrophus,* and *Azotobacter vinlandii;* and phytosphere yeast species such as *Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces roseus, S. odorus, Kluyveromyces veronae,* and *Aureobasidium pollulans.* Of particular interest are the pigmented microorganisms.

A wide variety of methods is available for introducing a *Bt* gene encoding a toxin into a microorganism host under conditions which allow for stable maintenance and expression of the gene. These methods are well known to those skilled in the art and are described, for example, in US Pat. No. 5135 867.

Treatment of cells. *Bacillus thuringiensis* or recombinant cells expressing the *Bt* toxins can be treated to prolong the toxin activity and stabilize the cell. The pesticide microcapsule that is formed comprises the *Bt* toxin or toxins within a cellular structure that has been stabilized and will protect the toxin when the microcapsule is applied to the environment of the target pest. Suitable host cells may include either prokaryotes or eukaryotes, normally being limited to those cells which do not produce substances toxic to higher organisms, such as mammals. However, organisms which produce substances toxic to higher organisms could be used, where the toxic substances are unstable or the level of application sufficiently low as to avoid any possibility of toxicity to a mammalian host. As hosts, of particular interest will be the prokaryotes and the lower eukaryotes, such as fungi.

The cell will usually be intact and be substantially in the proliferative form when treated, rather than in a spore form, although in some instances spores may be employed.

Treatment of the microbial cell, e.g., a microbe containing the B.t. toxin gene or genes, can be by chemical or physical means, or by a combination of chemical and/or physical means, so long as the technique does not deleteriously affect the properties of the toxin, nor diminish the cellular capability of protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as glutaraldehyde; anti-infectives, such as zephiran chloride and cetylpyridinium chloride; alcohols, such as isopropyl and ethanol; various histologic fixatives, such as Lugol iodine, Bouin's fixative, various acids and Helly's fixative (See: Humason, Gretchen L., Animal Tissue Techniques, W. H. Freeman and Company, 1967); or a combination of physical (heat) and chemical agents that preserve and prolong the activity of the toxin produced in the cell when the cell is administered to the host environment. Examples of physical means are short wavelength radiation such as gamma-radiation and X-radiation, freezing, UV irradiation, lyophilization, and the like. Methods for treatment of microbial cells are disclosed in U.S. Pat. Nos. 4,695,455 and 4,695,462.

The cells generally will have enhanced structural stability which will enhance resistance to environmental conditions. Where the pesticide is in a proform, the method of cell treatment should be selected so as not to inhibit processing of the proform to the mature form of the pesticide by the target pest pathogen. For example, formaldehyde will crosslink proteins and could inhibit processing of the proform of a polypeptide pesticide. The method of treatment should retain at least a substantial portion of the bio-availability or bioactivity of the toxin.

Characteristics of particular interest in selecting a host cell for purposes of production include ease of introducing the B.t. gene or genes into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; survival in aqueous environments; lack of mammalian toxicity; attractiveness to pests for ingestion; ease of killing and fixing without damage to the toxin; and the like. Other considerations include ease of formulation and handling, economics, storage stability, and the like.

Growth of cells. The cellular host containing the B.t. insecticidal gene or genes may be grown in any convenient nutrient medium, where the DNA construct provides a selective advantage, providing for a selective medium so that substantially all or all of the cells retain the B.t. gene. These cells may then be harvested in accordance with conventional ways. Alternatively, the cells can be treated prior to harvesting.

The B.t. cells producing the toxins of the invention can be cultured using standard art media and fermentation techniques. Upon completion of the fermentation cycle the bacteria can be harvested by first separating the B.t. spores and crystals from the fermentation broth by means well known in the art. The recovered B.t. spores and crystals can be formulated into a wettable powder, liquid concentrate, granules or other formulations by the addition of surfactants, dispersants, inert carriers, and other components to facilitate handling and application for particular target pests. These formulations and application procedures are all well known in the art.

Formulations. Formulated bait granules containing an attractant and spores, crystals, and toxins of the B.t. isolates, or recombinant microbes comprising the genes obtainable from the B.t. isolates disclosed herein, can be applied to the soil. Formulated product can also be applied as a seed-coating or root treatment or total plant treatment at later stages of the crop cycle. Plant and soil treatments of B.t. cells may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

As would be appreciated by a person skilled in the art, the pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about 10² to about 10⁴ cells/mg. These formulations will be administered at about 50 mg (liquid or dry) to 1 kg or more per hectare.

The formulations can be applied to the environment of the lepidopteran pest, e.g., foliage or soil, by spraying, dusting, sprinkling, or the like.

Plant transformation. A preferred recombinant host for production of the insecticidal proteins of the subject invention is a transformed plant. Genes encoding *Bt* toxin proteins, as disclosed herein, can be inserted into plant cells using a variety of techniques which are well known in the art. For example, a large number of cloning vectors comprising a replication system in *Escherichia coli* and a marker that permits selection of the transformed cells are available for preparation for the insertion of foreign genes into higher plants. The vectors comprise, for example, pBR322, pUC series, M13mp series, pACYC184, *inter alia.* Accordingly, the DNA fragment having the sequence encoding the *Bt* toxin protein can be inserted into the vector at a suitable restriction site. The resulting plasmid is used for transformation into *E. coli.* The *E. coli* cells are cultivated in a suitable nutrient medium, then harvested and lysed. The plasmid is recovered. Sequence analysis, restriction analysis, electrophoresis, and other biochemical-molecular biological methods are generally carried out as methods of analysis. After each manipulation, the DNA sequence used can be cleaved and joined to the next DNA sequence. Each plasmid sequence can be cloned in the same or other plasmids. Depending on the method of inserting desired genes into the plant, other DNA sequences may be necessary. If, for example, the Ti or Ri plasmid is used for the transformation of the plant cell, then at least the right border, but often the right and the left border of the Ti or Ri plasmid T-DNA, has to be joined as the flanking region of the genes to be inserted. The use of T-DNA for the transformation of plant cells has been intensively researched and sufficiently described in EP 120 516, Lee and Gelvin (2008), Hoekema (1985), Fraley *et al.,* (1986), and An *et al.,* (1985), and is well established in the art.

Once the inserted DNA has been integrated in the plant genome, it is relatively stable. The transformation vector normally contains a selectable marker that confers on the transformed plant cells resistance to a biocide or an antibiotic, such as Bialaphos, Kanamycin, G418, Bleomycin, or Hygromycin, *inter alia.* The individually employed marker should accordingly permit the selection of transformed cells rather than cells that do not contain the inserted DNA.

A large number of techniques is available for inserting DNA into a plant host cell. Those techniques include transformation with T-DNA using *Agrobacterium tumefaciens* or *Agrobacterium rhizogenes* as transformation agent, fusion, injection, biolistics (microparticle bombardment), or electroporation as well as other possible methods. If Agrobacteria are used for the transformation, the DNA to be inserted has to be cloned into special plasmids, namely either into an intermediate vector or into a binary vector. The intermediate vectors can be integrated into the Ti or Ri plasmid by homologous recombination owing to sequences that are homologous to sequences in the T-DNA. The Ti or Ri plasmid also comprises the vir region necessary for the transfer of the T-DNA. Intermediate vectors cannot replicate themselves in Agrobacteria. The intermediate vector can be transferred into *Agrobacterium tumefaciens* by means of a helper plasmid (conjugation). Binary vectors can replicate themselves both in *E. coli* and in Agrobacteria. They comprise a selection marker gene and a linker or polylinker which are framed by the Right and Left T-DNA border regions. They can be transformed directly into Agrobacteria (Holsters *et al.,* 1978). The *Agrobacterium* used as host cell is to comprise a plasmid carrying a vir region. The vir region is necessary for the transfer of the T-DNA into the plant cell. Additional T-DNA may be contained. The bacterium so transformed is used for the transformation of plant cells. Plant explants can advantageously be cultivated with *Agrobacterium tumefaciens* or *Agrobacterium rhizogenes* for the transfer of the DNA into the plant cell. Whole plants can then be regenerated from the infected plant material (for example, pieces of leaf, segments of stalk, roots, but also protoplasts or suspension-cultivated cells) in a suitable medium, which may contain antibiotics or biocides for selection. The plants so obtained can then be tested for the presence of the inserted DNA. No special demands are made of the plasmids in the case of injection and electroporation. It is possible to use ordinary plasmids, such as, for example, pUC derivatives.

The transformed cells grow inside the plants in the usual manner. They can form germ cells and transmit the transformed trait(s) to progeny plants. Such plants can be grown in the normal manner and crossed with plants that have the same transformed hereditary factors or other hereditary factors. The resulting hybrid individuals have the corresponding phenotypic properties.

Plants can be transformed with genes wherein the codon usage has been optimized for plants. See, for example, US Patent No. 5380831. While some truncated toxins are exemplified herein, it is well-known in the *Bt* art that 130 kDa-type (full-length) toxins have an N-terminal half that is the core toxin, and a C-terminal half that is the protoxin "tail." Thus, appropriate "tails" can be used with truncated / core toxins of the subject invention. *See e.g.* US Patent No. 6218188 and US Patent No. 6673990. In addition, methods for creating synthetic *Bt* genes for use in plants are known in the art (Stewart and Burgin, 2007). One non-limiting example of a preferred transformed plant is a fertile maize plant comprising a plant expressible gene encoding a Cry1Fa protein, and further comprising a second plant expressible gene encoding a Cry1Da protein.

Transfer (or introgression) of the Cry1Fa- and Cry1Da-determined trait(s) into inbred maize lines can be achieved by recurrent selection breeding, for example by backcrossing. In this case, a desired recurrent parent is first crossed to a donor inbred (the non-recurrent parent) that carries the appropriate gene(s) for the Cry1F- and Cry1D-determined traits. The progeny of this cross is then mated back to the recurrent parent followed by selection in the resultant progeny for the desired trait(s) to be transferred from the non-recurrent parent. After three, preferably four, more preferably five or more generations of backcrosses with the recurrent parent with selection for the desired trait(s), the progeny will be heterozygous for loci controlling the trait(s) being transferred, but will be like the recurrent parent for most or almost all other genes (see, for example, Poehlman & Sleper (1995) Breeding Field Crops, 4th Ed., 172-175; Fehr (1987) Principles of Cultivar Development, Vol. 1: Theory and Technique, 360-376).

Insect Resistance Management (IRM) Strategies. Roush *et al.,* for example, outlines two-toxin strategies, also called "pyramiding" or "stacking," for management of insecticidal transgenic crops. (The Royal Society. Phil. Trans. R. Soc. Lond. B. (1998) 353, 1777-1786).

On their website, the United States Environmental Protection Agency (epa.gov/oppbppd1/biopesticides/pips/bt_corn_refuge_2006.htm) publishes the following requirements for providing non-transgenic (*i.e., non-B.t.)* refuges (a section of non-Bt crops / corn) for use with transgenic crops producing a single Bt protein active against target pests.

"The specific structured requirements for corn borer-protected Bt (Cry1Ab or Cry1F) corn products are as follows:
Structured refuges: 20% non-Lepidopteran Bt corn refuge in Corn Belt; 50% non-Lepidopteran Bt refuge in Cotton Belt

Blocks
Internal (*i.e.,* within the Bt field)
External (*i.e.,* separate fields within ½ mile (¼ mile if possible) of the
Bt field to maximize random mating)

In-field Strips
Strips must be at least 4 rows wide (preferably 6 rows) to reduce the effects of larval movement"

In addition, the National Corn Growers Association, on their website: (ncga.com/insect-resistance-management-fact-sheet-bt-corn)
also provides similar guidance regarding the refuge requirements. For example:
"Requirements of the Corn Borer IRM:
- Plant at least 20% of your corn acres to refuge hybrids
- In cotton producing regions, refuge must be 50%
- Must be planted within 1/2 mile of the refuge hybrids
- Refuge can be planted as strips within the Bt field; the refuge strips must be at least 4 rows wide
- Refuge may be treated with conventional pesticides only if economic thresholds are reached for target insect
- Bt-based sprayable insecticides cannot be used on the refuge corn
- Appropriate refuge must be planted on every farm with Bt corn"

As stated by Roush *et al.* (on pages 1780 and 1784 right column, for example), stacking or pyramiding of two different proteins each effective against the target pests and with little or no cross-resistance can allow for use of a smaller refuge. Roush suggests that for a successful stack, a refuge size of less than 10% refuge, can provide comparable resistance management to about 50% refuge for a single (non-pyramided) trait. For currently available pyramided Bt corn products, the U.S. Environmental Protection Agency requires significantly less (generally 5%) structured refuge of non-Bt corn be planted than for single trait products (generally 20%).

There are various ways of providing the IRM effects of a refuge, including various geometric planting patterns in the fields (as mentioned above) and in-bag seed mixtures, as discussed further by Roush *et al.* (*supra*), and U.S. Patent No. 6,551,962.

The above percentages, or similar refuge ratios, can be used for the subject double or triple stacks or pyramids. For triple stacks with three modes of action against a single target pest, a goal would be zero refuge (or less than 5% refuge, for example). This is particularly true for commercial acreage - of over 10 acres for example.

Following are examples that illustrate procedures for practicing the invention. These examples should not be construed as limiting. All percentages are by weight and all solvent mixture proportions are by volume unless otherwise noted. All temperatures are in degrees Celsius.

Unless specifically indicated or implied, the terms "a", "an", and "the" signify "at least one" as used herein.

### EXAMPLE 1

### BIOASSAY DATA

Cry1Da expressed in transgenic corn (pDAS5163) provides protection from feeding by fall armyworm (FAW), *Spodoptera frugiperda* (J.E. Smith). The same events are more effective in controlling FAW that have developed resistance to CrylFa and are clearly superior to corn plants containing event TC1507, which is arguably the industry-leading insect resistance trait for FAW control.

We have also demonstrated that Cry1Fa (protein from recombinant *Pseudomonas fluorescens* strain DR1649; plasmid pDAB1817) and Cry1Da (protein from recombinant *Pseudomonas fluorescens* strain DC782) are both effective at controlling FAW in artificial diet bioassays and that the potency of the combination is greater than is expected from their individual potencies.

Based on the data described above, co-expressing Cry1Da and Cry1Fa can produce a high dose IRM stack for FAW, other important *Spodoptera* species, and perhaps other lepidopteran pests. Other proteins can be added to this combination to add spectrum. For example in corn, the addition of Cry1Ab would create an IRM stack for European corn borer (ECB), *Ostrinia nubilalis* (Hübner).

As shown in **Figure 1****,** damage (mean % leaf damage + SEM) to corn leaf segments infested with FAW (blue bars) or rFAW (purple bars). All treatments preceded by the numbers "5163" are leaf segments from plants transformed with a construct containing Cry1Da. Plants in which no Cry1Da expression was detected are grouped on the far left of the graph. Plants in which Cry1Da expression was detected are grouped in the center of the graph. Non-transgenic (i.e., negative) controls are on the far right of the graph and are labeled "B104", "Hill", and "Isoline". A commercial inbred containing Cry1Fa is the first treatment on right (labeled "Herculex I") and is the same genetic background as the non-transgenic control labeled "Isoline".

A protoxin chimera consisting of the coding sequence for the trypsin cleaved limit toxin of Cry1Da and the coding sequence for the c-terminal protoxin region of Cry1Ab was created and engineered into an expression cassette capable of directing expression in corn (pDAS5163). Corn was transformed using *Agrobacterium tumefacians* and events containing the Cry1Da/1Ab chimera were identified. Leaf sections from regenerated plants were bioassayed with wild type fall armyworm (FAW) or larvae from a fall armyworm population that was resistant to Cry1Fa (rFAW). Cry1Da/1Ab transformed plants did reduce feeding of FAW but were not as effective as the inbred containing 2 copies of Cry1Fa (**Figure 1**). (The Cry1Da events tested were hemizygous for the transgene while the converted inbred was homozygous for event TC1507.) In contrast, the same events containing Cry1Da/1Ab were generally much more effective in reducing the feeding of rFAW than the inbred containing Cry1Fa (**Figure 1**).

The insecticidal activity of Cry1Fa (protein from recombinant *Pseudomonas fluorescens* strain DR1649; plasmid pDAB1817), Cry1Da (protein from recombinant *P. fluorescens* strain DC782), and a 1:1 (w:w) combination of the 2 was tested in standard, artificial diet bioassays used to assess potency. Potency estimates were made using LOGIT analysis (JMP®8.0, SAS Inc. 2008) which produced LC₅₀ estimates and upper and lower limits (95%) for the LC₅₀. A test for synergism was conducted using the method described by Tabashnik (1992) by which an expected value for the potency of a combination is calculated using the potencies of each component alone. A combination is considered synergistic when the estimated upper confidence limit of the combination is lower than the calculated expected potency. In the case of fall armyworm (FAW) and a population of fall armyworm that was resistant to Cry1Fa (rFAW), the upper confidence limits for the LC₅₀s of the combination were lower than the estimated potencies (Tables 1 & 2) thereby leading to the conclusion that the combination of Cry1Fa and Cry1Da on these 2 populations is synergistic.

Table 1. Potency estimates, upper and lower limits of the 95% confidence interval (LCL and UCL, respectively), for Cry1Fa, Cry1Da, and the 1:1 (w;w) combination of the 2 on wild type fall armyworm (FAW), *Spodoptera frugiperda.* The last column contains the expected LC₅₀ value based on the potency of each protein alone using the formula described by Tabashnik (1992). Tabashnik BE. Evaluation of synergism among Bacillus thuringiensis toxins. Applied and Environmental Microbiology 58[10], 3343-3346. 1992. A combination is considered synergistic when the expected value is higher than the upper confidence limit for the combination.

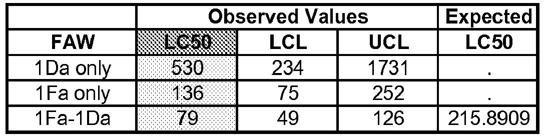

Table 2. Potency estimates, upper and lower limits of the 95% confidence interval (LCL and UCL, respectively), for Cry1Fa, Cry1Da, and the 1:1 (w;w) combination of the 2 on Cry1Fa-resistant fall armyworm (rFAW), *Spodoptera frugiperda.* The last column contains the expected LC₅₀ value based on the potency of each protein alone using the formula described by Tabashnik (1992). A combination is considered synergistic when the expected value is higher than the upper confidence limit for the combination.

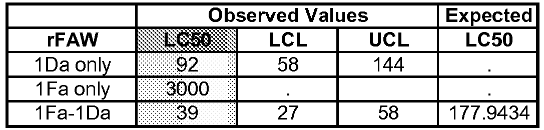

### EXAMPLE 2

### SUMMARY OF BINDING DATA

Competition binding experiments conducted with ¹²⁵I-labeled Cry1Da using brush border membrane vesicles (BBMV) isolated from FAW are described below. The results from these experiments demonstrate that Cry1Da binds tightly to its receptor and that Cry1Fa does not compete with Cry1Da for binding sites. If resistance to Cry1Da could be based on a mutation to the receptor observed in these studies, these data suggest that Cry1Fa would be a good IRM tool for managing such resistant populations or mitigating the development of such resistance. Results from bioassays with Cry1Fa-resitant FAW (rFAW) demonstrate that Cry1Da is active on this population. Together, these data suggest that Cry1Fa and Cry1Da could be an IRM stack that effectively mitigates the development of resistance to either insecticidal protein.

Receptor binding assays show that 125I Cry1Da binds tightly to its receptor(s), and can be effectively competed off by unlabeled Cry1Da. Neither Cry1Ab, Cry1Fa or Cry1Be can compete off 125I Cry1Da from its receptor site(s) in FAW BBMV's, indicating that Cry1Da has a unique binding site in the midgut of FAW that Cry1Ab, Cry1F and Cry1Be do not compete with. Since rFAW are as sensitive to Cry1Da as wild type FAW, this indicates that the putative receptor site that is altered in rFAW insects is not the receptor site that Cry1Da binds to. Thus, Cry1Da is an excellent stacking partner for Cry1Fa since it interacts at a different target site which is responsible for its biological activity.

When 125I Cry1Da was added to FAW BBMV's, only non-radiolabeled Cry1Da itself was able to displace the bound 125I Cry1Da. The inability of Cry1Fa, Cry1Ab, and Cry1Be to displace the bound 125I Cry1Da from the BBMV's indicated that in FAW midgut, Cry1Da bound to a unique receptor site that Cry1Fa, Cry1Ab, and Cry1Be do not interact, even though all four of these different Cry toxins are active against FAW larvae.

### EXAMPLE 3

### Design of chimeric toxins comprising Cry1 core toxins and heterologous protoxins

Chimeric Toxins. Chimeric proteins utilizing the core toxin domain of one Cry toxin fused to the protoxin segment of another Cry toxin have previously been reported, for example, in US Patent No. 5593881 and US Patent No. 5932209.

Cry1Da chimeric protein variants of this invention include chimeric toxins comprising an N-terminal core toxin segment derived from a Cry1Da insecticidal toxin fused to a heterologous delta endotoxin protoxin segment at some point past the end of the core toxin segment. The transition from the core toxin to the heterologous protoxin segment can occur at approximately the native core toxin/protoxin junction or, in the alternative, a portion of the native protoxin (extending past the core toxin segment) can be retained, with the transition to the heterologous protoxin occurring downstream. In variant fashion, the core toxin and protoxin segments may comprise exactly the amino acid sequence of the native toxins from which they are derived, or may include amino acid additions, deletions, or substitutions that do not diminish, and may enhance, the biological function of the segments when fused to one another.

For example, a chimeric toxin of the subject invention comprises a core toxin segment derived from Cry1Da and a heterologous protoxin. In a preferred embodiment of the invention, the core toxin segment derived from Cry1Da2 (594 amino acids) is fused to a heterologous segment comprising a protoxin segment derived from a Cry1Ab delta-endotoxin (545 amino acids). The 1139 amino acid sequence of the chimeric protein, herein referred to as Cry1Da. It is to be understood that other chimeric fusions comprising Cry1Da2 core toxin variants and protoxins derived from Cry1Ab are within the scope of this invention.

A second chimeric protein of the invention comprises a core toxin segment derived from Cry1Fa (603 amino acids) fused to a heterologous segment comprising a protoxin segment derived from a Cry1Ab delta-endotoxin (545 amino acids). The 1148 amino acid sequence of the chimeric protein, herein called Cry1Fa.

### EXAMPLE 4

### Construction of expression plasmids encoding chimeric proteins and expression in Pseudomonas

Standard cloning methods [as described in, for example, Sambrook *et al.,* (1989) and Ausubel *et al.,* (1995), and updates thereof] were used in the construction of *Pseudomonas fluorescens* (Pf) expression construct pDOW2848 engineered to produce a full-length Cry1Da chimeric protein. Protein production was performed in *Pseudomonas fluorescens* strain MB214 (a derivative of strain MB101; *P. fluorescens* biovar I), having an insertion of a modified *lac* operon as disclosed in US Patent No. 5169760. The basic cloning strategy entailed subcloning a DNA fragment encoding the Cry1Da protein into plasmid vectors, whereby it is placed under the expression control of the *Ptac* promoter and the rrnBT1T2 terminator from plasmid pKK223-3 (PL Pharmacia, Milwaukee, WI). One such plasmid was named pDOW2848 and the MB214 isolate harboring this plasmid is named Dpf150.

Growth and Expression Analysis in Shake Flasks Production of the Cry1Da protein for characterization and insect bioassay was accomplished by shake-flask-grown *P. fluorescens* strain Dpf150. Cry1Da protein production driven by the *Ptac* promoter was conducted as described previously in US Patent No. 5527883. Details of the microbiological manipulations are available in Squires *et al.,* (2004), US Patent Application 20060008877, US Patent Application 20080193974, and US Patent Application 20080058262.

Expression was induced by addition of isopropyl-β-D-1-thiogalactopyranoside (IPTG) after an initial incubation of 24 hours at 30° with shaking. Cultures were sampled at the time of induction and at various times post-induction. Cell density was measured by optical density at 600 nm (OD₆₀₀).

Cell Fractionation and SDS-PAGE Analysis of Shake Flask Samples At each sampling time, the cell density of samples was adjusted to OD₆₀₀ = 20 and 1 mL aliquots were centrifuged at 14000 x g for five minutes. The cell pellets were frozen at -80°. Soluble and insoluble fractions from frozen shake flask cell pellet samples were generated using EasyLyse™ Bacterial Protein Extraction Solution (EPICENTRE® Biotechnologies, Madison, WI). Each cell pellet was resuspended in 1 mL EasyLyse™ solution and further diluted 1:4 in lysis buffer and incubated with shaking at room temperature for 30 minutes. The lysate was centrifuged at 14,000 rpm for 20 minutes at 4° and the supernatant was recovered as the soluble fraction. The pellet (insoluble fraction) was then resuspended in an equal volume of phosphate buffered saline (PBS; 11.9 mM Na₂HPO₄, 137 mM NaCl, 2.7 mM KCl, pH7.4).

Samples were mixed 1:1 with 2X Laemmli sample buffer containing β-mercaptoethanol (Sambrook *et al., supra*.) and boiled for 5 minutes prior to loading onto Criterion XT Bis-Tris 12% gels (Bio-Rad Inc., Hercules, CA). Electrophoresis was performed in the recommended XT MOPS buffer. Gels were stained with Bio-Safe Coomassie Stain according to the manufacturer's (Bio-Rad) protocol and imaged using the Alpha Innotech Imaging system (San Leandro, CA).

Inclusion body preparation. Cry1Da protein inclusion body (IB) preparations were performed on cells from *P. fluorescens* fermentations that produced insoluble *Bt* insecticidal protein, as demonstrated by SDS-PAGE and MALDI-MS (Matrix Assisted Laser Desorption/Ionization Mass Spectrometry). *P. fluorescens* fermentation pellets were thawed in a 37° water bath. The cells were resuspended to 25% w/v in lysis buffer [50 mM Tris, pH 7.5, 200 mM NaCl, 20 mM EDTA disodium salt (Ethylenediaminetetraacetic acid), 1% Triton X-100, and 5 mM Dithiothreitol (DTT); 5 mL/L of bacterial protease inhibitor cocktail (Catalog # P8465; Sigma-Aldrich, St. Louis, MO) were added just prior to use]. The cells were suspended using a hand-held homogenizer at lowest setting (Tissue Tearor, BioSpec Products, Inc., Bartlesville, OK). Lysozyme (25 mg of Sigma L7651, from chicken egg white) was added to the cell suspension by mixing with a metal spatula, and the suspension was incubated at room temperature for one hour. The suspension was cooled on ice for 15 minutes, then sonicated using a Branson Sonifier 250 (two 1- minute sessions, at 50% duty cycle, 30% output). Cell lysis was checked by microscopy. An additional 25 mg of lysozyme were added if necessary, and the incubation and sonication were repeated. Following confirmation of cell lysis via microscopy, the lysate was centrifuged at 11,500 x g for 25 minutes (4°) to form the IB pellet, and the supernatant was discarded. The IB pellet was resuspended with 100 mL lysis buffer, homogenized with the hand-held mixer and centrifuged as above. The IB pellet was repeatedly washed by resuspension (in 50 mL lysis buffer), homogenization, sonication, and centrifugation until the supernatant became colorless and the IB pellet became firm and off-white in color. For the final wash, the IB pellet was resuspended in sterile-filtered (0.22 µm) distilled water containing 2 mM EDTA, and centrifuged. The final pellet was resuspended in sterile-filtered distilled water containing 2 mM EDTA, and stored in 1 mL aliquots at -80°.

SDS-PAGE analysis and quantitation of protein in IB preparations was done by thawing a 1 mL aliquot of IB pellet and diluting 1:20 with sterile-filtered distilled water. The diluted sample was then boiled with 4X reducing sample buffer [250 mM Tris, pH6.8, 40% glycerol (v/v), 0.4% Bromophenol Blue (w/v), 8% SDS (w/v) and 8% β-mercaptoethanol (v/v)] and loaded onto a Novex® 4-20% Tris-Glycine, 12+2 well gel (Invitrogen) run with 1X Tris/Glycine/SDS buffer (BioRad). The gel was run for 60 min at 200 volts then stained with Coomassie Blue (50% G-250/50% R-250 in 45% methanol, 10% acetic acid), and destained with 7% acetic acid, 5% methanol in distilled water. Quantification of target bands was done by comparing densitometric values for the bands against Bovine Serum Albumin (BSA) standard samples run on the same gel to generate a standard curve.

Solubilization of Inclusion Bodies. Six mL of Cry1Da inclusion body suspension from *Pf* clone DPf150 were centrifuged on the highest setting of an Eppendorf model 5415C microfuge (approximately 14,000 x g) to pellet the inclusions. The storage buffer supernatant was removed and replaced with 25 mL of 100 mM sodium carbonate buffer, pH11, in a 50 mL conical tube. Inclusions were resuspended using a pipette and vortexed to mix thoroughly. The tube was placed on a gently rocking platform at 4° overnight to extract the target protein. The extract was centrifuged at 30,000 x g for 30 min at 4°, and the resulting supernatant was concentrated 5-fold using an Amicon Ultra-15 regenerated cellulose centrifugal filter device (30,000 Molecular Weight Cutoff; Millipore). The sample buffer was then changed to 10 mM CAPS [3-(cyclohexamino)1-propanesulfonic acid] pH 10 using disposable PD-10 columns (GE Healthcare, Piscataway, NJ).

Solubilization and trypsin activation of Inclusion Body protein. In some instances, Cry1Da inclusion body suspension from Pf clone DPf150 was centrifuged on the highest setting of an Eppendorf model 5415C microfuge (approximately 14,000 x g) to pellet the inclusions. The storage buffer supernatant was removed and replaced with 100 mM CAPS, pH 11 to provide a protein concentration of approximately 50 mg/mL. The tube was rocked at room temperature for three hours to completely solubilize the protein. Trypsin was added at an amount equal to 5% to 10% (w:w, based on the initial weight of IB powder) and digestion was accomplished by incubation while rocking overnight at 4° or by rocking 90-120 minutes at room temperature. Insoluble material was removed by centrifugation at 10,000 x g for 15 minutes, and the supernatant was applied to a MonoQ anion exchange column (10 mm by 10 cm). Activated CrylDa protein was eluted (as determined by SDS-PAGE, see below) by a 0% to 100% 1 M NaCl gradient over 25 column volumes. Fractions containing the activated protein were pooled and, when necessary, concentrated to less than 10 mL using an Amicon Ultra-15 regenerated cellulose centrifugal filter device as above. The material was then passed through a Superdex 200 column (16 mm by 60 cm) in buffer containing 100 mM NaCl. 10% glycerol, 0.5% Tween-20 and 1 mM EDTA. It was determined by SDS-PAGE analysis that the activated (enzymatically truncated) protein elutes at 65 to 70 mL. Fractions containing the activated protein were pooled and concentrated using the centrifugal concentrator as above.

Gel electrophoresis. The concentrated protein preparations were prepared for electrophoresis by diluting 1:50 in NuPAGE® LDS sample buffer (Invitrogen) containing 5 mM DTT as a reducing agent and heated at 95° for 4 minutes. The sample was loaded in duplicate lanes of a 4-12% NuPAGE® gel alongside five BSA standards ranging from 0.2 µg to 2 µg/lane (for standard curve generation). Voltage was applied at 200 V using MOPS SDS running buffer (Invitrogen) until the tracking dye reached the bottom of the gel. The gel was stained with 0.2% Coomassie Blue G-250 in 45% methanol, 10% acetic acid, and destained, first briefly with 45% methanol, 10% acetic acid, and then at length with 7% acetic acid, 5% methanol until the background cleared. Following destaining, the gel was scanned with a BioRad Fluor-S MultiImager. The instrument's Quantity One Software v.4.5.2 was used to obtain background-subtracted volumes of the stained protein bands and to generate the BSA standard curve that was used to calculate the concentration of chimeric CrylDa protein in the stock solution.

### EXAMPLE 5

### Preparation of Cry1Fa and Cry1Da core toxin proteins and isolation of Spodoptera frugiperda brush border membrane vesicles for competitive binding experiments

The following Examples evaluate the competition binding of Cry1 core toxin proteins to putative receptors in insect gut tissues. It is shown that 125I-labeled Cry1Da core toxin protein binds with high affinity to Brush Border Membrane Vesicles (BBMV's) prepared from *Spodoptera frugiperda* (fall armyworm) and that Cry1Fa core toxin protein does not compete with this binding.

Purification of Cry Proteins. A gene encoding a chimeric Cry1Da protein was expressed in the *Pseudomonas fluorescens* expression strain as described in Example 4. In similar fashion, a gene encoding a chimeric protein comprising the Cry1Fa core toxin (603 amino acids) and Cry1Ab protoxin (545 amino acids) was expressed in the *Pf* system. In the Cry1Fa instance, the expression plasmid was named pDAB1817, and the *P. fluorescens* strain that harbors pDAB1817 was named DPf129. The proteins were purified by the methods of Example 4, and trypsin digestion to produce activated core toxins from the full-length proteins was then performed, and the products were purified by the methods described in Example 4. Preparations of the trypsin processed (activated core toxin) proteins were >95% pure and had a molecular weight of approximately 65 kDa as determined experimentally by SDS-PAGE. As used herein, the activated core toxin prepared from the Cry1Da protein is called the Cry1Da core toxin protein, and the activated core toxin prepared from the Cry1Fa protein is called the Cry1Fa core toxin protein.

Preparation and Fractionation of Solubilized BBMV's. Standard methods of protein quantification and SDS-polyacrylamide gel electrophoresis were employed as taught, for example, in Sambrook *et al.* (1989) and Ausubel *et al.* (1995), and updates thereof.

Last instar *S. frugiperda* larvae were fasted overnight and then dissected after chilling on ice for 15 minutes. The midgut tissue was removed from the body cavity, leaving behind the hindgut attached to the integument. The midgut was placed in a 9X volume of ice cold homogenization buffer (300 mM mannitol, 5 mM EGTA, 17 mM Tris base, pH7.5), supplemented with Protease Inhibitor Cocktail (Sigma-Aldrich P-2714) diluted as recommended by the supplier. The tissue was homogenized with 15 strokes of a glass tissue homogenizer. BBMV's were prepared by the MgCl₂ precipitation method of Wolfersberger (1993). Briefly, an equal volume of a 24 mM MgCl₂ solution in 300 mM mannitol was mixed with the midgut homogenate, stirred for 5 minutes and allowed to stand on ice for 15 min. The solution was centrifuged at 2,500 x g for 15 min at 4°. The supernatant was saved and the pellet suspended into the original volume of 0.5X diluted homogenization buffer and centrifuged again. The two supernatants were combined and centrifuged at 27,000 x g for 30 min at 4° to form the BBMV fraction. The pellet was suspended into BBMV Storage Buffer (10 mM HEPES, 130 mM KCl, 10% glycerol, pH7.4) to a protein concentration of about 3 mg/mL. Protein concentration was determined using Bovine Serum Albumin (BSA) as the standard. Alkaline phosphatase determination (a marker enzyme for the BBMV fraction) was made prior to freezing the samples using the QuantiChrom™ DALP-250 Alkaline Phosphatase Assay Kit (Gentaur Molecular Products, Kampenhout, BE) following the manufacturer's instructions. The specific activity of this enzyme typically increased 7-fold compared to that found in the starting midgut homogenate fraction. The BBMV's were aliquoted into 250 µL samples, flash frozen in liquid nitrogen and stored at -80°.

Electrophoresis. Analysis of proteins by SDS-PAGE was conducted under reducing (*i.e.* in 5% β-mercaptoethanol, BME) and denaturing (*i.e.* heated 5 minutes at 90° in the presence of 2% SDS) conditions. Proteins were loaded into wells of a 4% to 20% Tris-Glycine polyacrylamide gel (BioRad; Hercules, CA) and separated at 200 volts for 60 minutes. Protein bands were detected by staining with Coomassie Brilliant Blue R-250 (BioRad) for one hour, and destained with a solution of 5% methanol in 7% acetic acid. The gels were imaged and analyzed using a BioRad Fluro-S Multi Imager™. Relative molecular weights of the protein bands were determined by comparison to the mobilities of known molecular weight proteins observed in a sample of BenchMark™ Protein Ladder (Life Technologies, Rockville, MD) loaded into one well of the gel.

Iodination of Cry1Da core toxin protein. Purified Cry1Da core toxin protein was iodinated using Pierce Iodination Beads (Thermo Fisher Scientific, Rockford, IL). Briefly, two Iodination Beads were washed twice with 500 µL of PBS (20 mM sodium phosphate, 0.15 M NaCl, pH7.5), and placed into a 1.5 mL centrifuge tube with 100 µL of PBS. 0.5 mCi of 125I-labeled sodium iodide was added, the components were allowed to react for 5 minutes at room temperature, then 1 µg of Cry1Da core toxin protein was added to the solution and allowed to react for an additional 3 to 5 minutes. The reaction was terminated by pipetting the solution from the Iodination Beads and applying it to a Zeba™ spin column (Invitrogen) equilibrated in 50 mM CAPS, pH10.0, 1 mM DTT (dithiothreitol), 1 mM EDTA, and 5% glycerol. The Iodination Beads were washed twice with 10 µL of PBS and the wash solution was also applied to the Zeba™ desalting column. The radioactive solution was eluted through the spin column by centrifuging at 1,000 x g for 2 min. 125I-radiolabeled Cry1Da core toxin protein was then dialyzed against 50 mM CAPS, pH10.0, 1 mM DTT, 1 mM EDTA, and 5% glycerol.

Imaging. Radio-purity of the iodinated Cry1Da core toxin protein was determined by SDS-PAGE and phosphorimaging. Briefly, SDS-PAGE gels were dried using a BioRad gel drying apparatus following the manufacturer's instructions. The dried gels were imaged by wrapping them in Mylar film (12 µm thick) and exposing them under a Molecular Dynamics storage phosphor screen (35 cm x 43 cm) for 1 hour. The plates were developed using a Molecular Dynamics Storm 820 phosphorimager and the image was analyzed using ImageQuant™ software.

### EXAMPLE 6

### Binding of 125I-labeled Cry1 core toxin protein to BBMV's from Spodoptera frugiperda

A saturation curve was generated to determine the optimal amount of BBMV protein to use in the binding assays with Cry1Da and Cry1Fa core toxin proteins. 0.5 nM of 125I-radiolabeled Cry1 core toxin protein was incubated for 1 hr at 28° in binding buffer (8 mM NaHPO₄, 2 mM KH₂PO₄, 150 mM NaCl, 0.1% BSA, pH7.4) with amounts of BBMV protein ranging from 0 µg/mL to 500 µg/mL (total volume of 0.5 mL). 125I-labeled Cry1 core toxin protein bound to the BBMV proteins was separated from the unbound fraction by sampling 150 µL of the reaction mixture in triplicate into separate 1.5 mL centrifuge tubes and centrifuging the samples at 14,000 x g for 8 minutes at room temperature. The supernatant was gently removed and the pellet was washed three times with ice cold binding buffer. The bottom of the centrifuge tube containing the pellet was cut off, placed into a 13 x 75 mm glass culture tube and the samples were counted for 5 minutes each in the gamma counter. CPM (counts per minute) obtained minus background CPM (reaction with no BBMV protein) was plotted versus BBMV protein concentration. In accordance with results reported by others (Luo *et al.,* 1999), the optimal concentration of BBMV protein to use in the binding assays was determined to be 150 µg/mL.

### EXAMPLE 7

### Competitive binding assays to BBMVs from S. fruziperda with core toxin proteins of Cry1Da and Cry1Fa

Homologous and heterologous competition binding assays were conducted using 150 µg/mL of *S. frugiperda* BBMV protein and 0.5 nM of the 125I-radiolabeled Cry1Da core toxin protein. Concentrations of the competitive non-radiolabeled Cry1Fa core toxin protein added to the reaction mixture ranged from 0.045 nM to 1000 nM and were added at the same time as the radioactive Cry1Da core toxin protein, to assure true binding competition. Incubations were carried out for 1 hr at 28° and the amount of 125I-labeled Cry1Da core toxin protein bound to the BBMV (specific binding) was measured as described above. Non-specific binding was represented by the counts obtained in the presence of 1,000 nM of non-radiolabeled Cry1Da core toxin protein. One hundred percent total binding was considered to be the amount of binding in the absence of any competitor Cry1Fa core toxin protein.

Receptor binding assays using 125I-labeled Cry1Da core toxin protein determined the ability of the Cry1Fa core toxin protein to displace this radiolabeled ligand from its binding site on BBMV's from *S. frugiperda.* The results show that the Cry1Fa core toxin protein did not displace bound 125I-labeled Cry1Da core toxin protein from its receptor protein(s) at concentrations as high as 1000 nM (2000 times the concentration of the radioactive binding ligand). As expected, unlabeled Cry1Da core toxin protein was able to displace radiolabeled Cry1Da core toxin protein from its binding protein(s), exhibiting a sigmoidal dose response curve with 50% displacement occurring at 5 nM.

It is thus indicated that the Cry1Da core toxin protein interacts with a binding site in *S. frugiperda* BBMV that does not bind the Cry1Fa core toxin protein.

### References

Finney, D.J. 1971. Probit analysis. Cambridge University Press, England.
Hua, G., L. Masson, J. L. Jurat-Fuentes, G. Schwab, and M. J. Adang. Binding analyses of Bacillus thuringiensis Cry d-endotoxins using brush border membrane vesicles of Ostrinia nubilalis. Applied and Environmental Microbiology 67[2], 872-879. 2001.
LeOra Software. 1987. POLO-PC. A user's guide to probit and logit analysis. Berkeley, CA.
McGaughey, W. H., F. Gould, and W. Gelernter. Bt resistance management. Nature Biotechnology 16[2], 144-146. 1998
Marçon, P.R.G.C., L.J. Young, K. Steffey, and B.D. Siegfried. 1999. Baseline susceptibility of the European corn borer, Ostrinia nubilalis (Hübner) (Lepidoptera: Pyralidae) to Bacillus thuringiensis toxins. J. Econ. Entomol. 92 (2): 280-285.
Robertson, L.J. and H.K. Preisler. 1992. Pesticide bioassays with arthropods. CRC Press, Boca Ranton, FL.
SAS Institute Inc. 1988. SAS procedures guide, Release 6.03 edition. SAS Institute Inc, Cary, NC.
Stone, B.F. 1968. A formula for determining degree of dominance in cases of monofactorial inheritance of resistance to chemicals. Bull. WHO 38:325-329.
Van Mellaert, H., J. Botterman, J. Van Rie, and H. Joos. Transgenic plants for the prevention of development of insects resistant to Bacillus thuringiensis toxins. (Plant Genetic Systems N.V., Belg. 89-401499[400246], 57-19901205. EP. 5-31-1989

### Appendix A

### List of delta-endotoxins - from Crickmore et al. website (cited in application) Accession Number is to NCBI entry (if available)

| **Name** | **Acc No.** | **Authors** | **Year** | **Source Strain** | **Comment** |
|---|---|---|---|---|---|
| Cry1Aa1 | AAA22353 | Schnepf et al | 1985 | Bt kurstaki HD1 | |
| Cry1Aa2 | AAA22552 | Shibano et al | 1985 | Bt sotto | |
| Cry1Aa3 | BAA00257 | Shimizu et al | 1988 | Bt aizawai IPL7 | |
| Cry1Aa4 | CAA31886 | Masson et al | 1989 | Bt entomocidus | |
| Cry1Aa5 | BAA04468 | Udayasuriyan et al | 1994 | Bt Fu-2-7 | |
| Cry1Aa6 | AAA86265 | Masson et al | 1994 | Bt kurstaki NRD-12 | |
| Cry1Aa7 | AAD46139 | Osman et al | 1999 | Bt C12 | |
| Cry1Aa8 | I26149 | Liu | 1996 | | DNA sequence only |
| Cry1Aa9 | BAA77213 | Nagamatsu et al | 1999 | Bt dendrolimus T84A1 | |
| Cry1Aa10 | AAD55382 | Hou and Chen | 1999 | Bt kurstaki HD-1-02 | |
| Cry1Aa11 | CAA70856 | Tounsi et al | 1999 | Bt kurstaki | |
| Cry1Aa12 | AAP80146 | Yao et al | 2001 | Bt Ly30 | |
| Cry1Aa13 | AAM44305 | Zhong et al | 2002 | Bt sotto | |
| Cry1Aa14 | AAP40639 | Ren et al | 2002 | unpublished | |
| Cry1Aa15 | AAY66993 | Sauka et al | 2005 | Bt INTA Mol-12 | |
| Cry1Ab1 | AAA22330 | Wabiko et al | 1986 | Bt berliner 1715 | |
| Cry1Ab2 | AAA22613 | Thorne et al | 1986 | Bt kurstaki | |
| Cry1Ab3 | AAA22561 | Geiser et al | 1986 | Bt kurstaki HD1 | |
| Cry1Ab4 | BAA00071 | Kondo et al | 1987 | Bt kurstaki HD1 | |
| Cry1Ab5 | CAA28405 | Hofte et al | 1986 | Bt berliner 1715 | |
| Cry1Ab6 | AAA22420 | Hefford et al | 1987 | Bt kurstaki NRD-12 | |
| Cry1Ab7 | CAA31620 | Haider & Ellar | 1988 | Bt aizawai IC1 | |
| Cry1Ab8 | AAA22551 | Oeda et al | 1987 | Bt aizawai IPL7 | |
| Cry1Ab9 | CAA38701 | Chak & Jen | 1993 | Bt aizawai HD133 | |
| Cry1Ab10 | A29125 | Fischhoff et al | 1987 | Bt kurstaki HD1 | |
| Cry1Ab11 | I12419 | Ely & Tippett | 1995 | Bt A20 | DNA sequence only |
| Cry1Ab12 | AAC64003 | Silva-Werneck et al | 1998 | Bt kurstaki S93 | |
| Cry1Ab13 | AAN76494 | Tan et al | 2002 | Bt c005 | |
| Cry1Ab14 | AAG16877 | Meza-Basso & Theoduloz | 2000 | Native Chilean Bt | |
| Cry1Ab15 | AAO13302 | Li et al | 2001 | Bt B-Hm-16 | |
| Cry1Ab16 | AAK55546 | Yu et al | 2002 | Bt AC-11 | |
| Cry1Ab17 | AAT46415 | Huang et al | 2004 | Bt WB9 | |
| Cry1Ab18 | AAQ88259 | Stobdan et al | 2004 | Bt | |
| Cry1Ab19 | AAW31761 | Zhong et al | 2005 | Bt X-2 | |
| Cry1Ab20 | ABB72460 | Liu et al | 2006 | BtC008 | |
| Cry1Ab21 | ABS18384 | Swiecicka et al | 2007 | Bt IS5056 | |
| Cry1Ab22 | ABW87320 | Wu and Feng | 2008 | BtS2491Ab | |
| Cry1Ab-like | AAK14336 | Nagarathinam et al | 2001 | Bt kunthala RX24 | uncertain sequence |
| Cry1Ab-like | AAK14337 | Nagarathinam et al | 2001 | Bt kunthala RX28 | uncertain sequence |
| Cry1Ab-like | AAK14338 | Nagarathinam et al | 2001 | Bt kunthala RX27 | uncertain sequence |
| Cry1Ab-like | ABG88858 | Lin et al | 2006 | Bt ly4a3 | insufficient sequence |
| Cry1Ac1 | AAA22331 | Adang et al | 1985 | Bt kurstaki HD73 | |
| Cry1Ac2 | AAA22338 | Von Tersch et al | 1991 | Bt kenyae | |
| Cry1Ac3 | CAA38098 | Dardenne et al | 1990 | Bt BTS89A | |
| Cry1Ac4 | AAA73077 | Feitelson | 1991 | Bt kurstaki PS85A1 | |
| Cry1Ac5 | AAA22339 | Feitelson | 1992 | Bt kurstaki PS81GG | |
| Cry1Ac6 | AAA86266 | Masson et al | 1994 | Bt kurstaki NRD-12 | |
| Cry1Ac7 | AAB46989 | Herrera et al | 1994 | Bt kurstaki HD73 | |
| Cry1Ac8 | AAC44841 | Omolo et al | 1997 | Bt kurstaki HD73 | |
| Cry1Ac9 | AAB49768 | Gleave et al | 1992 | Bt DSIR732 | |
| Cry1Ac10 | CAA05505 | Sun | 1997 | Bt kurstaki YBT-1520 | |
| Cry1Ac11 | CAA10270 | Makhdoom & Riazuddin | 1998 | | |
| Cry1Ac12 | I12418 | Ely & Tippett | 1995 | Bt A20 | DNA sequence only |
| Cry1Ac13 | AAD38701 | Qiao et al | 1999 | Bt kurstaki HD1 | |
| Cry1Ac14 | AAQ06607 | Yao et al | 2002 | Bt Ly30 | |
| Cry1Ac15 | AAN07788 | Tzeng et al | 2001 | Bt from Taiwan | |
| Cry1Ac16 | AAU87037 | Zhao et al | 2005 | Bt H3 | |
| Cry1Ac17 | AAX18704 | Hire et al | 2005 | Bt kenyae HD549 | |
| Cry1Ac18 | AAY88347 | Kaur & Allam | 2005 | Bt SK-729 | |
| Cry1Ac19 | ABD37053 | Gao et al | 2005 | Bt C-33 | |
| Cry1Ac20 | ABB89046 | Tan et al | 2005 | | |
| Cry1Ac21 | AAY66992 | Sauka et al | 2005 | INTA Mol-12 | |
| Cry1Ac22 | ABZ01836 | Zhang & Fang | 2008 | Bt W015-1 | |
| Cry1Ac23 | CAQ30431 | Kashyap et al | 2008 | Bt | |
| Cry1Ac24 | ABL01535 | Arango et al | 2008 | Bt 146-158-01 | |
| Cry1Ac25 | FJ513324 | Guan Peng et al | 2008 | Bt Tm37-6 | No NCBI link July 09 |
| Cry1Ac26 | FJ617446 | Guan Peng et al | 2009 | Bt Tm41-4 | No NCBI link July 09 |
| Cry1Ac27 | FJ617447 | Guan Peng et al | 2009 | Bt Tm44-1B | No NCBI link July 09 |
| Cry1Ac28 | ACM90319 | Li et al | 2009 | Bt Q-12 | |
| Cry1Ad1 | AAA22340 | Feitelson | 1993 | Bt aizawai PS81I | |
| Cry1Ad2 | CAA01880 | Anonymous | 1995 | Bt PS81RR1 | |
| Cry1Ae1 | AAA22410 | Lee & Aronson | 1991 | Bt alesti | |
| Cry1Af1 | AAB82749 | Kang et al | 1997 | Bt NT0423 | |
| Cry1Ag1 | AAD46137 | Mustafa | 1999 | | |
| Cry1Ah1 | AAQ14326 | Tan et al | 2000 | | |
| Cry1Ah2 | ABB76664 | Qi et al | 2005 | Bt alesti | |
| Cry1Ai1 | AAO39719 | Wang et al | 2002 | | |
| Cry1A-like | AAK14339 | Nagarathinam et al | 2001 | Bt kunthala nags3 | uncertain sequence |
| Cry1Ba1 | CAA29898 | Brizzard & Whiteley | 1988 | Bt thuringiensis HD2 | |
| Cry1Ba2 | CAA65003 | Soetaert | 1996 | Bt entomocidus HD110 | |
| Cry1Ba3 | AAK63251 | Zhang et al | 2001 | | |
| Cry1Ba4 | AAK51084 | Nathan et al | 2001 | Bt entomocidus HD9 | |
| Cry1Ba5 | ABO20894 | Song et al | 2007 | Bt sfw-12 | |
| Cry1Ba6 | ABL60921 | Martins et al | 2006 | Bt S601 | |
| Cry1Bb1 | AAA22344 | Donovan et al | 1994 | Bt EG5847 | |
| Cry1Bc1 | CAA86568 | Bishop et al | 1994 | Bt morrisoni | |
| Cry1Bd1 | AAD10292 | Kuo et al | 2000 | Bt wuhanensis HD525 | |
| Cry1Bd2 | AAM93496 | Isakova et al | 2002 | Bt 834 | |
| Cry1Be1 | AAC32850 | Payne et al | 1998 | BtPS158C2 | |
| Cry1Be2 | AAQ52387 | Baum et al | 2003 | | |
| Cry1Be3 | FJ716102 | Xiaodong Sun et al | 2009 | Bt | No NCBI link July 09 |
| Cry1Bf1 | CAC50778 | Arnaut et al | 2001 | | |
| Cry1Bf2 | AAQ52380 | Baum et al | 2003 | | |
| Cry1Bg1 | AAO39720 | Wang et al | 2002 | | |
| Cry1Ca1 | CAA30396 | Honee et al | 1988 | Bt entomocidus 60.5 | |
| Cry1Ca2 | CAA31951 | Sanchis et al | 1989 | Bt aizawai 7.29 | |
| Cry1Ca3 | AAA22343 | Feitelson | 1993 | Bt aizawai PS81I | |
| Cry1Ca4 | CAA01886 | Van Mellaert et al | 1990 | Bt entomocidus HD110 | |
| Cry1Ca5 | CAA65457 | Strizhov | 1996 | Bt aizawai 7.29 | |
| Cry1Ca6 | AAF37224 | Yu et al | 2000 | Bt AF-2 | |
| Cry1Ca7 | AAG50438 | Aixing et al | 2000 | BtJ8 | |
| Cry1Ca8 | AAM00264 | Chen et al | 2001 | Bt c002 | |
| Cry1Ca9 | AAL79362 | Kao et al | 2003 | Bt G10-01A | |
| Cry1Ca10 | AAN16462 | Lin et al | 2003 | Bt E05-20a | |
| Cry1Ca11 | AAX53094 | Cai et al | 2005 | Bt C-33 | |
| Cry1Cb1 | M97880 | Kalman et al | 1993 | Bt galleriae HD29 | DNA sequence only |
| Cry1Cb2 | AAG35409 | Song et al | 2000 | Bt c001 | |
| Cry1Cb3 | ACD50894 | Huang et al | 2008 | Bt 087 | |
| Cry1Cb- like | AAX63901 | Thammasittirong et al | 2005 | Bt TA476-1 | insufficient sequence |
| Cry1Da1 | CAA38099 | Hofte et al | 1990 | Bt aizawai HD68 | |
| Cry1Da2 | I76415 | Payne & Sick | 1997 | | DNA sequence only |
| Cry1Db1 | CAA80234 | Lambert | 1993 | Bt BTS00349A | |
| Cry1Db2 | AAK48937 | Li et al | 2001 | Bt B-Pr-88 | |
| Cry1Dc1 | ABK35074 | Lertwiriyawong et al | 2006 | Bt JC291 | |
| Cry1Ea1 | CAA37933 | Visser et al | 1990 | Bt kenyae 4F1 | |
| Cry1Ea2 | CAA39609 | Bosse et al | 1990 | Bt kenyae | |
| Cry1Ea3 | AAA22345 | Feitelson | 1991 | Bt kenyae PS81F | |
| Cry1Ea4 | AAD04732 | Barboza-Corona et al | 1998 | Bt kenyae LBIT-147 | |
| Cry1Ea5 | A15535 | Botterman et al | 1994 | | DNA sequence only |
| Cry1Ea6 | AAL50330 | Sun et al | 1999 | Bt YBT-032 | |
| Cry1Ea7 | AAW72936 | Huehne et al | 2005 | Bt JC190 | |
| Cry1Ea8 | ABX11258 | Huang et al | 2007 | Bt HZM2 | |
| Cry1Eb1 | AAA22346 | Feitelson | 1993 | Bt aizawai PS81A2 | |
| Cry1Fa1 | AAA22348 | Chambers et al | 1991 | Bt aizawai EG6346 | |
| Cry1Fa2 | AAA22347 | Feitelson | 1993 | Bt aizawai PS81I | |
| Cry1Fb1 | CAA80235 | Lambert | 1993 | Bt BTS00349A | |
| Cry1Fb2 | BAA25298 | Masuda & Asano | 1998 | Bt morrisoni INA67 | |
| Cry1Fb3 | AAF21767 | Song et al | 1998 | Bt morrisoni | |
| Cry1Fb4 | AAC10641 | Payne et al | 1997 | | |
| Cry1Fb5 | AAO13295 | Li et al | 2001 | Bt B-Pr-88 | |
| Cry1Fb6 | ACD50892 | Huang et al | 2008 | Bt 012 | |
| Cry1Fb7 | ACD50893 | Huang et al | 2008 | Bt 087 | |
| Cry1Ga1 | CAA80233 | Lambert | 1993 | Bt BTS0349A | |
| Cry1Ga2 | CAA70506 | Shevelev et al | 1997 | Bt wuhanensis | |
| Cry1Gb1 | AAD10291 | Kuo & Chak | 1999 | Bt wuhanensis HD525 | |
| Cry1Gb2 | AAO13756 | Li et al | 2000 | Bt B-Pr-88 | |
| Cry1Gc | AAQ52381 | Baum et al | 2003 | | |
| Cry1Ha1 | CAA80236 | Lambert | 1993 | Bt BTS02069AA | |
| Cry1Hb1 | AAA79694 | Koo et al | 1995 | Bt morrisoni BF190 | |
| Cry1H-like | AAF01213 | Srifah et al | 1999 | Bt JC291 | insufficient sequence |
| Cry1Ia1 | CAA44633 | Tailor et al | 1992 | Bt kurstaki | |
| Cry1Ia2 | AAA22354 | Gleave et al | 1993 | Bt kurstaki | |
| Cry1Ia3 | AAC36999 | Shin et al | 1995 | Bt kurstaki HD1 | |
| Cry1Ia4 | AAB00958 | Kostichka et al | 1996 | Bt AB88 | |
| Cry1Ia5 | CAA70124 | Selvapandiyan | 1996 | Bt 61 | |
| Cry1Ia6 | AAC26910 | Zhong et al | 1998 | Bt kurstaki S101 | |
| Cry1Ia7 | AAM73516 | Porcar et al | 2000 | Bt | |
| Cry1Ia8 | AAK66742 | Song et al | 2001 | | |
| Cry1Ia9 | AAQ08616 | Yao et al | 2002 | Bt Ly30 | |
| Cry1Ia10 | AAP86782 | Espindola et al | 2003 | Bt thuringiensis | |
| Cry1Ia11 | CAC85964 | Tounsi et al | 2003 | Bt kurstaki BNS3 | |
| Cry1Ia12 | AAV53390 | Grossi de Sa et al | 2005 | Bt | |
| Cry1Ia13 | ABF83202 | Martins et al | 2006 | Bt | |
| Cry1Ia14 | ACG63871 | Liu & Guo | 2008 | Bt11 | |
| Cry1Ia15 | FJ617445 | Guan Peng et al | 2009 | Bt E-1B | No NCBI link July 2009 |
| Cry1Ia16 | FJ617448 | Guan Peng et al | 2009 | Bt E-1A | No NCBI link July 2009 |
| Cry1Ib1 | AAA82114 | Shin et al | 1995 | Bt entomocidus BP465 | |
| Cry1Ib2 | ABW88019 | Guan et al | 2007 | Bt PP61 | |
| Cry1Ib3 | ACD75515 | Liu & Guo | 2008 | Bt GS8 | |
| Cry1Ic1 | AAC62933 | Osman et al | 1998 | Bt C18 | |
| Cry1Ic2 | AAE71691 | Osman et al | 2001 | | |
| Cry1Id1 | AAD44366 | Choi | 2000 | | |
| Cry1Ie1 | AAG43526 | Song et al | 2000 | Bt BTC007 | |
| Cry1If1 | AAQ52382 | Baum et al | 2003 | | |
| Cry1I-like | AAC31094 | Payne et al | 1998 | | insufficient sequence |
| Cry1I-like | ABG88859 | Lin & Fang | 2006 | Bt ly4a3 | insufficient sequence |
| Cry1Ja1 | AAA22341 | Donovan | 1994 | Bt EG5847 | |
| Cry1Jb1 | AAA98959 | Von Tersch & Gonzalez | 1994 | Bt EG5092 | |
| Cry1Jc1 | AAC31092 | Payne et al | 1998 | | |
| Cry1Jc2 | AAQ52372 | Baum et al | 2003 | | |
| Cry1Jd1 | CAC50779 | Arnaut et al | 2001 | Bt | |
| Cry1Ka1 | AAB00376 | Koo et al | 1995 | Bt morrisoni BF190 | |
| Cry1La1 | AAS60191 | Je et al | 2004 | Bt kurstaki K1 | |
| Cry1-like | AAC31091 | Payne et al | 1998 | | insufficient sequence |
| Cry2Aa1 | AAA22335 | Donovan et al | 1989 | Bt kurstaki | |
| Cry2Aa2 | AAA83516 | Widner & Whiteley | 1989 | Bt kurstaki HD1 | |
| Cry2Aa3 | D86064 | Sasaki et al | 1997 | Bt sotto | DNA sequence only |
| Cry2Aa4 | AAC04867 | Misra et al | 1998 | Bt kenyae HD549 | |
| Cry2Aa5 | CAA10671 | Yu & Pang | 1999 | Bt SL39 | |
| Cry2Aa6 | CAA10672 | Yu & Pang | 1999 | Bt YZ71 | |
| Cry2Aa7 | CAA10670 | Yu & Pang | 1999 | Bt CY29 | |
| Cry2Aa8 | AAO13734 | Wei et al | 2000 | Bt Dongbei 66 | |
| Cry2Aa9 | AAO13750 | Zhang et al | 2000 | | |
| Cry2Aa10 | AAQ04263 | Yao et al | 2001 | | |
| Cry2Aa11 | AAQ52384 | Baum et al | 2003 | | |
| Cry2Aa12 | ABI83671 | Tan et al | 2006 | Bt Rpp39 | |
| Cry2Aa13 | ABL01536 | Arango et al | 2008 | Bt 146-158-01 | |
| Cry2Aa14 | ACF04939 | Hire et al | 2008 | Bt HD-550 | |
| Cry2Ab1 | AAA22342 | Widner & Whiteley | 1989 | Bt kurstaki HD1 | |
| Cry2Ab2 | CAA39075 | Dankocsik et al | 1990 | Bt kurstaki HD1 | |
| Cry2Ab3 | AAG36762 | Chen et al | 1999 | Bt BTC002 | |
| Cry2Ab4 | AAO13296 | Li et al | 2001 | Bt B-Pr-88 | |
| Cry2Ab5 | AAQ04609 | Yao et al | 2001 | Bt ly30 | |
| Cry2Ab6 | AAP59457 | Wang et al | 2003 | Bt WZ-7 | |
| Cry2Ab7 | AAZ66347 | Udayasuriyan et al | 2005 | Bt 14-1 | |
| Cry2Ab8 | ABC95996 | Huang et al | 2006 | Bt WB2 | |
| Cry2Ab9 | ABC74968 | Zhang et al | 2005 | Bt LLB6 | |
| Cry2Ab10 | EF157306 | Lin et al | 2006 | Bt LyD | |
| Cry2Ab11 | CAM84575 | Saleem et al | 2007 | Bt CMBL-BT1 | |
| Cry2Ab12 | ABM21764 | Lin et al | 2007 | Bt LyD | |
| Cry2Ab13 | ACG76120 | Zhu et al | 2008 | Bt ywc5-4 | |
| Cry2Ab14 | ACG76121 | Zhu et al | 2008 | Bt Bts | |
| Cry2Ac1 | CAA40536 | Aronson | 1991 | Bt shanghai S1 | |
| Cry2Ac2 | AAG35410 | Song et al | 2000 | | |
| Cry2Ac3 | AAQ52385 | Baum et al | 2003 | | |
| Cry2Ac4 | ABC95997 | Huang et al | 2006 | Bt WB9 | |
| Cry2Ac5 | ABC74969 | Zhang et al | 2005 | | |
| Cry2Ac6 | ABC74793 | Xia et al | 2006 | Bt wuhanensis | |
| Cry2Ac7 | CAL18690 | Saleem et al | 2008 | Bt SBSBT-1 | |
| Cry2Ac8 | CAM09325 | Saleem et al | 2007 | Bt CMBL-BT1 | |
| Cry2Ac9 | CAM09326 | Saleem et al | 2007 | Bt CMBL-BT2 | |
| Cry2Ac10 | ABN15104 | Bai et al | 2007 | Bt QCL-1 | |
| Cry2Ac11 | CAM83895 | Saleem et al | 2007 | Bt HD29 | |
| Cry2Ac12 | CAM83896 | Saleem et al | 2007 | Bt CMBL-BT3 | |
| Cry2Ad1 | AAF09583 | Choi et al | 1999 | Bt BR30 | |
| Cry2Ad2 | ABC86927 | Huang et al | 2006 | Bt WB10 | |
| Cry2Ad3 | CAK29504 | Saleem et al | 2006 | Bt5_2AcT(1) | |
| Cry2Ad4 | CAM32331 | Saleem et al | 2007 | Bt CMBL-BT2 | |
| Cry2Ad5 | CAO78739 | Saleem et al | 2007 | Bt HD29 | |
| Cry2Ae1 | AAQ52362 | Baum et al | 2003 | | |
| Cry2Af1 | ABO30519 | Beard et al | 2007 | Bt C81 | |
| Cry2Ag | ACH91610 | Zhu et al | 2008 | Bt JF19-2 | |
| Cry2Ah | EU939453 | Zhang et al | 2008 | Bt | No NCBI link July 09 |
| Crv2Ah2 | ACL80665 | Zhang et al | 2009 | Bt BRC-ZQL3 | |
| Cry2Ai | FJ788388 | Udayasuriyan et al | 2009 | Bt | No NCBI link July 09 |
| Crv3Aa1 | AAA22336 | Herrnstadt et al | 1987 | Bt san diego | |
| Cry3Aa2 | AAA22541 | Sekar et al | 1987 | Bt tenebrionis | |
| Cry3Aa3 | CAA68482 | Hofte et al | 1987 | | |
| Cry3Aa4 | AAA22542 | McPherson et al | 1988 | Bt tenebrionis | |
| Cry3Aa5 | AAA50255 | Donovan et al | 1988 | Bt morrisoni EG2158 | |
| Cry3Aa6 | AAC43266 | Adams et al | 1994 | Bt tenebrionis | |
| Cry3Aa7 | CAB41411 | Zhang et al | 1999 | Bt 22 | |
| Cry3Aa8 | AAS79487 | Gao and Cai | 2004 | Bt YM-03 | |
| Cry3Aa9 | AAW05659 | Bulla and Candas | 2004 | Bt UTD-001 | |
| Cry3Aa10 | AAU29411 | Chen et al | 2004 | Bt 886 | |
| Cry3Aa11 | AAW82872 | Kurt et al | 2005 | Bt tenebrionis Mm2 | |
| Cry3Aa12 | ABY49136 | Sezen et al | 2008 | Bt tenebrionis | |
| Cry3Ba1 | CAA34983 | Sick et al | 1990 | Bt tolworthi 43F | |
| Cry3Ba2 | CAA00645 | Peferoen et al | 1990 | Bt PGSI208 | |
| Cry3Bb1 | AAA22334 | Donovan et al | 1992 | Bt EG4961 | |
| Cry3Bb2 | AAA74198 | Donovan et al | 1995 | Bt EG5144 | |
| Cry3Bb3 | I15475 | Peferoen et al | 1995 | | DNA sequence only |
| Cry3Ca1 | CAA42469 | Lambert et al | 1992 | Bt kurstaki BtI109P | |
| Cry4Aa1 | CAA68485 | Ward & Ellar | 1987 | Bt israelensis | |
| Cry4Aa2 | BAA00179 | Sen et al | 1988 | Bt israelensis HD522 | |
| Cry4Aa3 | CAD30148 | Berry et al | 2002 | Bt israelensis | |
| Cry4A-like | AAY96321 | Mahalakshmi et al | 2005 | Bt LDC-9 | insufficient sequence |
| Cry4Ba1 | CAA30312 | Chungjatpornchai et al | 1988 | Bt israelensis 4Q2-72 | |
| Cry4Ba2 | CAA30114 | Tungpradubkul et al | 1988 | Bt israelensis | |
| Cry4Ba3 | AAA22337 | Yamamoto et al | 1988 | Bt israelensis | |
| Cry4Ba4 | BAA00178 | Sen et al | 1988 | Bt israelensis HD522 | |
| Cry4Ba5 | CAD30095 | Berry et al | 2002 | Bt israelensis | |
| Cry4Ba-like | ABC47686 | Mahalakshmi et al | 2005 | Bt LDC-9 | insufficient sequence |
| Cry4Ca1 | EU646202 | Shu et al | 2008 | | No NCBI link July 09 |
| Cry4Cb1 | FJ403208 | Jun & Furong | 2008 | Bt HS18-1 | No NCBI link July 09 |
| Cry4Cb2 | FJ597622 | Jun & Furong | 2008 | Bt Ywc2-8 | No NCBI link July 09 |
| Cry4Cc1 | FJ403207 | Jun & Furong | 2008 | Bt MC28 | No NCBI link July 09 |
| Cry5Aa1 | AAA67694 | Narva et al | 1994 | Bt darmstadiensis PS17 | |
| Cry5Ab1 | AAA67693 | Narva et al | 1991 | Bt darmstadiensis PS17 | |
| Cry5Ac1 | 134543 | Payne et al | 1997 | | DNA sequence only |
| Cry5Ad1 | ABQ82087 | Lenane et al | 2007 | Bt L366 | |
| Cry5Ba1 | AAA68598 | Foncerrada & Narva | 1997 | Bt PS86Q3 | |
| Cry5Ba2 | ABW88932 | Guo et al | 2008 | YBT 1518 | |
| Cry6Aa1 | AAA22357 | Narva et al | 1993 | Bt PS52A1 | |
| Cry6Aa2 | AAM46849 | Bai et al | 2001 | YBT 1518 | |
| Cry6Aa3 | ABH03377 | Jia et al | 2006 | Bt 96418 | |
| Cry6Ba1 | AAA22358 | Narva et al | 1991 | Bt PS69D1 | |
| Cry7Aa1 | AAA22351 | Lambert et al | 1992 | Bt galleriae PGSI245 | |
| Cry7Ab1 | AAA21120 | Narva & Fu | 1994 | Bt dakota HD511 | |
| Cry7Ab2 | AAA21121 | Narva & Fu | 1994 | Bt kumamotoensis 867 | |
| Cry7Ab3 | ABX24522 | Song et al | 2008 | Bt WZ-9 | |
| Cry7Ab4 | EU380678 | Shu et al | 2008 | Bt | No NCBI link July 09 |
| Cry7Ab5 | ABX79555 | Aguirre-Arzola et al | 2008 | Bt monterrey GM-33 | |
| Cry7Ab6 | ACI44005 | Deng et al | 2008 | Bt HQ122 | |
| Cry7Ab7 | FJ940776 | Wang et al | 2009 | | No NCBI link Sept 09 |
| Cry7Ab8 | GU145299 | Feng Jing | 2009 | | No NCBI link Nov 09 |
| Cry7Ba1 | ABB70817 | Zhang et al | 2006 | Bt huazhongensis | |
| Cry7Ca1 | ABR67863 | Gao et al | 2007 | Bt BTH-13 | |
| Cry7Da1 | ACQ99547 | Yi et al | 2009 | Bt LH-2 | |
| Cry8Aa1 | AAA21117 | Narva & Fu | 1992 | Bt kumamotoensis | |
| Cry8Ab1 | EU044830 | Cheng et al | 2007 | Bt B-JJX | No NCBI link July 09 |
| Cry8Ba1 | AAA21118 | Narva & Fu | 1993 | Bt kumamotoensis | |
| Cry8Bb1 | CAD57542 | Abad et al | 2002 | | |
| Cry8Bc1 | CAD57543 | Abad et al | 2002 | | |
| Cry8Ca1 | AAA21119 | Satoetal. | 1995 | Bt japonensis Buibui | |
| Cry8Ca2 | AAR98783 | Shu et al | 2004 | BtHBF-1 | |
| Cry8Ca3 | EU625349 | Du et al | 2008 | Bt FTL-23 | No NCBI link July 09 |
| Cry8Da1 | BAC07226 | Asano et al | 2002 | Bt galleriae | |
| Cry8Da2 | BD133574 | Asano et al | 2002 | Bt | DNA sequence only |
| Cry8Da3 | BD133575 | Asano et al | 2002 | Bt | DNA sequence only |
| Cry8Db1 | BAF93483 | Yamaguchi et al | 2007 | Bt BBT2-5 | |
| Cry8Ea1 | AAQ73470 | Fuping et al | 2003 | Bt 185 | |
| Cry8Ea2 | EU047597 | Liu et al | 2007 | Bt B-DLL | No NCBI link July 09 |
| Cry8Fa1 | AAT48690 | Shu et al | 2004 | Bt 185 | also AAW81032 |
| Cry8Ga1 | AAT46073 | Shu et al | 2004 | Bt HBF-18 | |
| Cry8Ga2 | ABC42043 | Yan et al | 2008 | Bt 145 | |
| Cry8Ga3 | FJ198072 | Xiaodong et al | 2008 | Bt FCD114 | No NCBI link July 09 |
| Cry8Ha1 | EF465532 | Fuping et al | 2006 | Bt 185 | No NCBI link July 09 |
| Cry8Ia1 | EU381044 | Yan et al | 2008 | Bt su4 | No NCBI link July 09 |
| Cry8Ja1 | EU625348 | Du et al | 2008 | Bt FPT-2 | No NCBI link July 09 |
| Cry8Ka1 | FJ422558 | Quezado et al | 2008 | | No NCBI link July 09 |
| Cry8Ka2 | ACN87262 | Noguera & Ibarra | 2009 | Bt kenyae | |
| Cry8-like | FJ770571 | Noguera & Ibarra | 2009 | Bt canadensis | DNA sequence only |
| Cry8-like | ABS53003 | Mangena et al | 2007 | Bt | |
| Cry9Aa1 | CAA41122 | Shevelev et al | 1991 | Bt galleriae | |
| Cry9Aa2 | CAA41425 | Gleave et al | 1992 | Bt DSIR517 | |
| Cry9Aa3 | GQ249293 | Su et al | 2009 | Bt SC5(D2) | No NCBI link July 09 |
| Cry9Aa4 | GQ249294 | Su et al | 2009 | BtT03C001 | No NCBI link July 09 |
| Cry9Aa like | AAQ52376 | Baum et al | 2003 | | incomplete sequence |
| Cry9Ba1 | CAA52927 | Shevelev et al | 1993 | Bt galleriae | |
| Cry9Bb1 | AAV28716 | Silva-Werneck et al | 2004 | Btjaponensis | |
| Cry9Ca1 | CAA85764 | Lambert et al | 1996 | Bt tolworthi | |
| Cry9Ca2 | AAQ52375 | Baum et al | 2003 | | |
| Cry9Da1 | BAA19948 | Asano | 1997 | Bt japonensis N141 | |
| Cry9Da2 | AAB97923 | Wasano & Ohba | 1998 | Btjaponensis | |
| Cry9Da3 | GQ249295 | Su et al | 2009 | Bt T03B001 | No NCBI link July 09 |
| Cry9Da4 | GQ249297 | Su et al | 2009 | Bt T03B001 | No NCBI link July 09 |
| Cry9Db1 | AAX78439 | Flannagan & Abad | 2005 | Bt kurstaki DP 1019 | |
| Cry9Ea1 | BAA34908 | Midoh & Oyama | 1998 | Bt aizawai SSK-10 | |
| Cry9Ea2 | AAO12908 | Li et al | 2001 | Bt B-Hm-16 | |
| Cry9Ea3 | ABM21765 | Lin et al | 2006 | Bt lyA | |
| Cry9Ea4 | ACE88267 | Zhu et al | 2008 | Bt ywc5-4 | |
| Cry9Ea5 | ACF04743 | Zhu et al | 2008 | Bts | |
| Cry9Ea6 | ACG63872 | Liu & Guo | 2008 | Bt 11 | |
| Cry9Ea7 | FJ380927 | Sun et al | 2008 | | No NCBI link July 09 |
| Cry9Ea8 | GQ249292 | Su et al | 2009 | GQ249292 | No NCBI link July 09 |
| Cry9Eb1 | CAC50780 | Arnaut et al | 2001 | | |
| Cry9Eb2 | GQ249298 | Su et al | 2009 | Bt T03B001 | No NCBI link July 09 |
| Cry9Ec1 | AAC63366 | Wasano et al | 2003 | Bt galleriae | |
| Crv9Ed1 | AAX78440 | Flannagan & Abad | 2005 | Bt kurstaki DP 1019 | |
| Cry9Ee1 | GQ249296 | Su et al | 2009 | Bt T03B001 | No NCBI link Aug 09 |
| Cry9-like | AAC63366 | Wasano et al | 1998 | Bt galleriae | insufficient sequence |
| Cry10Aa1 | AAA22614 | Thorne et al | 1986 | Bt israelensis | |
| Cry10Aa2 | E00614 | Aran & Toomasu | 1996 | Bt israelensis ONR-60A | DNA sequence only |
| Cry10Aa3 | CAD30098 | Berry et al | 2002 | Bt israelensis | |
| Cry10A-like | DQ167578 | Mahalakshmi et al | 2006 | Bt LDC-9 | incomplete sequence |
| Cry11Aa1 | AAA22352 | Donovan et al | 1988 | Bt israelensis | |
| Cry11Aa2 | AAA22611 | Adams et al | 1989 | Bt israelensis | |
| Cry11Aa3 | CAD30081 | Berry et al | 2002 | Bt israelensis | |
| Cry11Aa-like | DQ166531 | Mahalakshmi et al | 2007 | Bt LDC-9 | incomplete sequence |
| Cry11Ba1 | CAA60504 | Delecluse et al | 1995 | Bt jegathesan 367 | |
| Cry11Bb1 | AAC97162 | Orduz et al | 1998 | Bt medellin | |
| Cry12Aa1 | AAA22355 | Narva et al | 1991 | Bt PS33F2 | |
| Cry13Aa1 | AAA22356 | Narva et al | 1992 | Bt PS63B | |
| Cry14Aa1 | AAA21516 | Narva et al | 1994 | Bt sotto PS80JJ1 | |
| Cry15Aa1 | AAA22333 | Brown & Whiteley | 1992 | Bt thompsoni | |
| Cry16Aa1 | CAA63860 | Barloy et al | 1996 | Cb malaysia CH18 | |
| Cry17Aa1 | CAA67841 | Barloy et al | 1998 | Cb malaysia CH18 | |
| Cry18Aa1 | CAA67506 | Zhang et al | 1997 | Paenibacillus popilliae | |
| Cry18Ba1 | AAF89667 | Patel et al | 1999 | Paenibacillus popilliae | |
| Cry18Ca1 | AAF89668 | Patel et al | 1999 | Paenibacillus popilliae | |
| Cry19Aa1 | CAA68875 | Rosso & Delecluse | 1996 | Bt jegathesan 367 | |
| Cry19Ba1 | BAA32397 | Hwang et al | 1998 | Bt higo | |
| Cry20Aa1 | AAB93476 | Lee & Gill | 1997 | Bt fukuokaensis | |
| Cry20Ba1 | ACS93601 | Noguera & Ibarra | 2009 | Bt higo LBIT-976 | |
| Cry20-like | GQ144333 | Yi et al | 2009 | Bt Y-5 | DNA sequence only |
| Cry21Aa1 | I32932 | Payne et al | 1996 | | DNA sequence only |
| Cry21Aa2 | I66477 | Feitelson | 1997 | | DNA sequence only |
| Cry21Ba1 | BAC06484 | Sato & Asano | 2002 | Bt roskildiensis | |
| Cry22Aa1 | 134547 | Payne et al | 1997 | | DNA sequence only |
| Cry22Aa2 | CAD43579 | Isaac et al | 2002 | Bt | |
| Cry22Aa3 | ACD93211 | Du et al | 2008 | Bt FZ-4 | |
| Cry22Ab1 | AAK50456 | Baum et al | 2000 | Bt EG4140 | |
| Cry22Ab2 | CAD43577 | Isaac et al | 2002 | Bt | |
| Cry22Ba1 | CAD43578 | Isaac et al | 2002 | Bt | |
| Cry23Aa1 | AAF76375 | Donovan et al | 2000 | Bt | Binary with Cry37Aa1 |
| Cry24Aa1 | AAC61891 | Kawalek and Gill | 1998 | Bt jegathesan | |
| Cry24Ba1 | BAD32657 | Ohgushi et al | 2004 | Bt sotto | |
| Cry24Ca1 | CAJ43600 | Beron & Salerno | 2005 | Bt FCC-41 | |
| Cry25Aa1 | AAC61892 | Kawalek and Gill | 1998 | Bt jegathesan | |
| Cry26Aa1 | AAD25075 | Wojciechowska et al | 1999 | Bt finitimus B-1166 | |
| Cry27Aa1 | BAA82796 | Saitoh | 1999 | Bt higo | |
| Cry28Aa1 | AAD24189 | Wojciechowska et al | 1999 | Bt finitimus B-1161 | |
| Crv28Aa2 | AAG00235 | Moore and Debro | 2000 | Bt finitimus | |
| Cry29Aa1 | CAC80985 | Delecluse et al | 2000 | Bt medellin | |
| Cry30Aa1 | CAC80986 | Delecluse et al | 2000 | Bt medellin | |
| Cry30Ba1 | BAD00052 | Ito et al | 2003 | Bt entomocidus | |
| Cry30Ca1 | BAD67157 | Ohgushi et al | 2004 | Bt sotto | |
| Cry30Ca2 | ACU24781 | Sun and Park | 2009 | Bt jegathesan 367 | |
| Cry30Da1 | EF095955 | Shu et al | 2006 | Bt Y41 | No NCBI link July09 |
| Cry30Db1 | BAE80088 | Kishida et al | 2006 | Bt aizawai BUN1-14 | |
| Cry30Ea1 | ACC95445 | Fang et al | 2007 | Bt S2160-1 | |
| Cry30Ea2 | FJ499389 | Jun et al | 2008 | Bt Ywc2-8 | No NCBI link July09 |
| Cry30Fa1 | ACI22625 | Tan et al | 2008 | Bt MC28 | |
| Cry30Ga1 | ACG60020 | Zhu et al | 2008 | Bt HS18-1 | |
| Cry31Aa1 | BAB11757 | Saitoh & Mizuki | 2000 | Bt 84-HS-1-11 | |
| Cry31Aa2 | AAL87458 | Jung and Cote | 2000 | Bt M15 | |
| Cry31Aa3 | BAE79808 | Uemori et al | 2006 | Bt B0195 | |
| Cry31Aa4 | BAF32571 | Yasutake et al | 2006 | Bt 79-25 | |
| Cry31Aa5 | BAF32572 | Yasutake et al | 2006 | Bt 92-10 | |
| Cry31Ab1 | BAE79809 | Uemori et al | 2006 | Bt B0195 | |
| Cry31Ab2 | BAF32570 | Yasutake et al | 2006 | Bt 31-5 | |
| Cry31Ac1 | BAF34368 | Yasutake et al | 2006 | Bt 87-29 | |
| Cry32Aa1 | AAG36711 | Balasubramanian al et | 2001 | Bt yunnanensis | |
| Cry32Ba1 | BAB78601 | Takebe et al | 2001 | Bt | |
| Cry32Ca1 | BAB78602 | Takebe et al | 2001 | Bt | |
| Cry32Da1 | BAB78603 | Takebe et al | 2001 | Bt | |
| Cry33Aa1 | AAL26871 | Kim et al | 2001 | Bt dakota | |
| Cry34Aa1 | AAG50341 | Ellis et al | 2001 | Bt PS80JJ1 | Binary with Cry35Aa1 |
| Cry34Aa2 | AAK64560 | Rupar et al | 2001 | Bt EG5899 | Binary with Cry35Aa2 |
| Cry34Aa3 | AAT29032 | Schnepf et al | 2004 | Bt PS69Q | Binary with Cry35Aa3 |
| Cry34Aa4 | AAT29030 | Schnepf et al | 2004 | Bt PS 185GG | Binary with Cry35Aa4 |
| Cry34Ab1 | AAG41671 | Moellenbeck et al | 2001 | Bt PS149B1 | Binary with Cry35Ab1 |
| Cry34Ac1 | AAG50118 | Ellis et al | 2001 | Bt PS167H2 | Binary with Cry35Ac1 |
| Cry34Ac2 | AAK64562 | Rupar et al | 2001 | Bt EG9444 | Binary with Cry35Ab2 |
| Cry34Ac3 | AAT29029 | Schnepf et al | 2004 | Bt KR1369 | Binary with Cry35Ab3 |
| Cry34Ba1 | AAK64565 | Rupar et al | 2001 | Bt EG4851 | Binary with Cry35Ba1 |
| Cry34Ba2 | AAT29033 | Schnepf et al | 2004 | Bt PS201L3 | Binary with Cry35Ba2 |
| Cry34Ba3 | AAT29031 | Schnepf et al | 2004 | Bt PS201HH2 | Binary with Cry35Ba3 |
| Cry35Aa1 | AAG50342 | Ellis et al | 2001 | Bt PS80JJ1 | Binary with Cry34Aa1 |
| Cry35Aa2 | AAK64561 | Rupar et al | 2001 | Bt EG5899 | Binary with Cry34Aa2 |
| Cry35Aa3 | AAT29028 | Schnepf et al | 2004 | Bt PS69Q | Binary with Cry34Aa3 |
| Cry35Aa4 | AAT29025 | Schnepf et al | 2004 | Bt PS185GG | Binary with Cry34Aa4 |
| Cry35Ab1 | AAG41672 | Moellenbeck et al | 2001 | Bt PS149B1 | Binary with Cry34Ab1 |
| Cry35Ab2 | AAK64563 | Rupar et al | 2001 | Bt EG9444 | Binary with Cry34Ac2 |
| Cry35Ab3 | AY536891 | AAT29024 | 2004 | Bt KR1369 | Binary with Cry34Ab3 |
| Cry35Ac1 | AAG50117 | Ellis et al | 2001 | Bt PS167H2 | Binary with Cry34Ac1 |
| Cry35Ba1 | AAK64566 | Rupar et al | 2001 | Bt EG4851 | Binary with Cry34Ba1 |
| Cry35Ba2 | AAT29027 | Schnepf et al | 2004 | Bt PS201L3 | Binary with Cry34Ba2 |
| Cry35Ba3 | AAT29026 | Schnepf et al | 2004 | Bt PS201HH2 | Binary with Cry34Ba3 |
| Cry36Aa1 | AAK64558 | Rupar et al | 2001 | Bt | |
| Cry37Aa1 | AAF76376 | Donovan et al | 2000 | Bt | Binary with Cry23Aa |
| Cry38Aa1 | AAK64559 | Rupar et al | 2000 | Bt | |
| Cry39Aa1 | BAB72016 | Ito et al | 2001 | Bt aizawai | |
| Cry40Aa1 | BAB72018 | Ito et al | 2001 | Bt aizawai | |
| Cry40Ba1 | BAC77648 | Ito et al | 2003 | Bun1-14 | |
| Cry40Ca1 | EU381045 | Shu et al | 2008 | Bt Y41 | No NCBI link July09 |
| Cry40Da1 | ACF15199 | Zhang et al | 2008 | Bt S2096-2 | |
| Cry41Aa1 | BAD35157 | Yamashita et al | 2003 | Bt A1462 | |
| Cry41Ab1 | BAD35163 | Yamashita et al | 2003 | Bt A1462 | |
| Cry42Aa1 | BAD35166 | Yamashita et al | 2003 | Bt A1462 | |
| Cry43Aa1 | BAD15301 | Yokoyama and Tanaka | 2003 | P. lentimorbus semadara | |
| Cry43Aa2 | BAD95474 | Nozawa | 2004 | P. popilliae popilliae | |
| Cry43Ba1 | BAD15303 | Yokoyama and Tanaka | 2003 | P. lentimorbus semadara | |
| Cry43-like | BAD15305 | Yokoyama and Tanaka | 2003 | P. lentimorbus semadara | |
| Cry44Aa | BAD08532 | Ito et al | 2004 | Bt entomocidus INA288 | |
| Cry45Aa | BAD22577 | Okumura et al | 2004 | Bt 89-T-34-22 | |
| Cry46Aa | BAC79010 | Ito et al | 2004 | Bt dakota | |
| Cry46Aa2 | BAG68906 | Ishikawa et al | 2008 | Bt A1470 | |
| Cry46Ab | BAD35170 | Yamagiwa et al | 2004 | Bt | |
| Cry47Aa | AAY24695 | Kongsuwan et al | 2005 | Bt CAA890 | |
| Cry48Aa | CAJ18351 | Jones and Berry | 2005 | Bs IAB59 | binary with 49Aa |
| Cry48Aa2 | CAJ86545 | Jones and Berry | 2006 | Bs 47-6B | binary with 49Aa2 |
| Cry48Aa3 | CAJ86546 | Jones and Berry | 2006 | Bs NHA15b | binary with 49Aa3 |
| Cry48Ab | CAJ86548 | Jones and Berry | 2006 | Bs LP1G | binary with 49Ab1 |
| Cry48Ab2 | CAJ86549 | Jones and Berry | 2006 | Bs 2173 | binary with 49Aa4 |
| Cry49Aa | CAH56541 | Jones and Berry | 2005 | Bs IAB59 | binary with 48Aa |
| Cry49Aa2 | CAJ86541 | Jones and Berry | 2006 | Bs 47-6B | binary with 48Aa2 |
| Cry49Aa3 | CAJ86543 | Jones and Berry | 2006 | BsNHA15b | binary with 48Aa3 |
| Cry49Aa4 | CAJ86544 | Jones and Berry | 2006 | Bs 2173 | binary with 48Ab2 |
| Cry49Ab1 | CAJ86542 | Jones and Berry | 2006 | Bs LP1G | binary with 48Ab1 |
| Cry50Aa1 | BAE86999 | Ohgushi et al | 2006 | Bt sotto | |
| Cry51Aa1 | ABI14444 | Meng et al | 2006 | Bt F14-1 | |
| Cry52Aa1 | EF613489 | Song et al | 2007 | Bt Y41 | No NCBI link July09 |
| Cry52Ba1 | FJ361760 | Jun et al | 2008 | Bt BM59-2 | No NCBI link July09 |
| Cry53Aa1 | EF633476 | Song et al | 2007 | Bt Y41 | No NCBI link July09 |
| Cry53Ab1 | FJ361759 | Jun et al | 2008 | Bt MC28 | No NCBI link July09 |
| Cry54Aa1 | ACA52194 | Tan et al | 2009 | Bt MC28 | |
| Cry55Aa1 | ABW88931 | Guo et al | 2008 | YBT 1518 | |
| Cry55Aa2 | AAE33526 | Bradfisch et al | 2000 | BT Y41 | |
| Cry56Aa1 | FJ597621 | Jun & Furong | 2008 | Bt Ywc2-8 | No NCBI link July09 |
| Cry56Aa2 | GQ483512 | Guan Peng et al | 2009 | Bt G7-1 | No NCBI link Aug09 |
| Cry57Aa1 | ANC87261 | Noguera & Ibarra | 2009 | Bt kim | |
| Cry58Aa1 | ANC87260 | Noguera & Ibarra | 2009 | Bt entomocidus | |
| Cry59Aa1 | ACR43758 | Noguera & Ibarra | 2009 | Bt kim LBIT-980 | |

| | | | | | | | |
|---|---|---|---|---|---|---|---|
| **Vip3Aa1** | Vip3Aa | AAC37036 | Estruch et al | 1996 | PNAS93, 5389-5394 | AB88 | |
| Vip3Aa2 | | AAC37037 | Estruch et al | | PNAS 93, 5389-5394 | AB424 | |
| Vip3Aa3 | Vip3Ac | | Estruch et al | 2000 | US 6137033 Oct 2000 | | |
| Vip3Aa4 | PS36A Sup | AAR81079 | Feitelson et al | 1998 | US 6656908 Dec 2003 | Bt PS36A | WO9818932(A 2,A3) 7 May 1998 |
| Vip3Aa5 | PS81F Sup | AAR81080 | Feitelson et al | 1998 | US 6656908 Dec 2003 | Bt PS81F | WO9818932(A 2,A3) 7 May 1998 |
| Vip3Aa6 | Jav90 Sup | AAR81081 | Feitelson et al | 1998 | US 6656908 Dec 2003 | Bt | WO9818932(A 2,A3) 7 May 1998 |
| Vip3Aa7 | Vip83 | AAK95326 | Cai et al | 2001 | unpublished | Bt YBT-833 | |
| Vip3Aa8 | Vip3A | AAK97481 | Loguercio et al | 2001 | unpublished | Bt HD125 | |
| | | CAA76665 | Selvapandiyan et al | | unpublished | | |
| Vip3Aa10 | Vip3V | AAN60738 | Doss et al | 2002 | Protein Expr. Purif. 26, 82-88 | | |
| Vip3Aa11 | Vip3A | AAR36859 | Liu et al | 2003 | unpublished | Bt C9 | |
| Vip3Aa12 | Vip3A-WB5 | AAM22456 | Wu and Guan | 2003 | unpublished | Bt | |
| Vip3Aa13 | Vip3A | AAL69542 | Chen et al | 2002 | Sheng Wu Gong Cheng Xue Bao 18, 687-692 | Bt S184 | |
| Vip3Aa14 | Vip | AAQ12340 | Polumetla et al | 2003 | unpublished | Bt tolworthi | |
| Vip3Aa15 | Vip3A | AAP51131 | Wu et al | 2004 | unpublished | Bt WB50 | |
| Vip3Aa16 | Vip3LB | AAW65132 | Mesrati et al | 2005 | FEMS Micro Lett 244, 353-358 | Bt | |
| Vip3Aa17 | Jav90 | | Feitelson et al | 1999 | US 6603063 Aug 2003 | Javelin 1990 | WO9957282(A2,A3) 11Nov 1999 |
| Vip3Aa18 | | AAX49395 | Cai and Xiao | 2005 | unpublished | Bt 9816C | |
| Vip3Aa19 | Vip3ALD | DQ241674 | Liu et al | 2006 | unpublished | Bt AL | |
| Vip3Aa19 | Vip3A-1 | DQ539887 | Hart et al | 2006 | unpublished | | |
| Vip3Aa20 | Vip3A-2 | DQ539888 | Hart et al | 2006 | unpublished | | |
| Vip3Aa21 | Vip | ABD84410 | Panbangred | 2006 | unpublished | Bt aizawai | |
| Vip3Aa22 | Vip3A-LS1 | | Lu et al | 2005 | unpublished | Bt LS1 | |
| Vip3Aa23 | Vip3A-LS8 | AAY41428 | Lu et al | 2005 | unpublished | Bt LS8 | |
| Vip3Aa24 | | BI 880913 | Song et al | 2007 | unpublished | Bt WZ-7 | |
| Vip3Aa25 | | EF608501 | Hsieh et al | 2007 | unpublished | | |
| Vip3Aa26 | | EU294496 | Shen and Guo | 2007 | unpublished | Bt TF9 | |
| Vip3Aa27 | | EU332167 | Shen and Guo | 2007 | unpublished | Bt 16 | |
| Vip3Aa28 | | FJ494817 | Xiumei Yu | 2008 | unpublished | Bt JF23-8 | |
| Vip3Aa29 | | FJ626674 | Xieumei et al | 2009 | unpublished | Bt JF21-1 | |
| Vip3Aa30 | | FJ626675 | Xieumei et al | 2009 | unpublished | MD2-1 | |
| Vip3Aa31 | | FJ626676 | Xieumei et al | 2009 | unpublished | JF21-1 | |
| Vip3Aa32 | | FJ626677 | Xieumei et al | 2009 | unpublished | MD2-1 | |
| . | | | | . | | | |
| **Vip3Ab1** | Vip3B | AAR40284 | Feitelson et al | 1999 | US 6603063 Aug 2003 | Bt KB59A4-6 | WO9957282(A 2,A3) 11Nov 1999 |
| Vip3Ab2 | Vip3D | AAY88247 | Feng and Shen | 2006 | unpublished | Bt | |
| . | | | | . | | | |
| **Vip3Ac1** | PS49C | | Narva et al | . | US application 2004012871 6 | | |
| . | | | | . | | | |
| **Vip3Ad1** | PS158C2 | | Narva et al | . | US application 2004012871 6 | | |
| Vip3Ad2 | ISP3B | CAI43276 | Van Rie et al | 2005 | unpublished | Bt | |
| | | | | . | | | |
| **Vip3Ae1** | ISP3C | CAI43277 | Van Rie et al | 2005 | unpublished | Bt | |
| . | | | | . | | | |
| **Vip3Af1** | ISP3A | CAI43275 | Van Rie et al | 2005 | unpublished | Bt | |
| Vip3Af2 | Vip3C | ADN08753 | Syngenta | . | WO 03/075655 | | |
| . | | | | . | | | |
| **Vip3Ag1** | Vip3B | ADN08758 | Syngenta | . | WO 02/078437 | | |
| Vip3Ag2 | | FJ556803 | Audtho et al | 2008 | | Bt | |
| | | | | . | | | |
| **Vip3Ah1** | Vip3S | DQ832323 | Li and Shen | 2006 | unpublished | Bt | |
| | | | | | | | |
| **Vip3Ba1** | | AAV70653 | Rang et al | 2004 | unpublished | | |
| | | | | | | | |
| **Vip3Bb1** | Vip3Z | ADN08760 | Syngenta | . | WO 03/075655 | | |
| Vip3Bb2 | | EF439819 | Akhurst et al | 2007 | | | |

## Claims

1. A transgenic plant or plant cell comprising DNA encoding and expressing a Cry1Da insecticidal protein and DNA encoding and expressing a Cry1Fa insecticidal protein.

2. Seed of a plant of claim 1, wherein said seed comprises DNA encoding and expressing a Cry1Da insecticidal protein and DNA encoding and expressing a CrylFa insecticidal protein.

3. A mixture of seeds comprising refuge seeds from non-Bt refuge plants, and a plurality of seeds of claim 2 comprising DNA encoding and expressing a Cry1Da insecticidal protein and DNA encoding and expressing a CrylFa insecticidal protein, wherein said refuge seeds comprise less than 40% of all the seeds in the mixture.

4. A method of managing development of resistance to a Cry toxin by an insect, said method comprising planting seeds comprising DNA encoding and expressing a Cry1Da insecticidal protein and DNA encoding and expressing a CrylFa insecticidal protein to produce a field of plants.

5. The transgenic plant of claim 1, said plant further comprising DNA encoding a CrylAb core toxin-containing protein, wherein a CrylAb core toxin is the N-terminal, insecticidally active, toxin portion of the CrylAb protein.

6. Use of a composition for controlling lepidopteran pests comprising cells that express effective amounts of both a CrylFa core toxin-containing protein and a Cry1 Da core toxin-containing protein, wherein a CrylFa core toxin is the N-terminal, insecticidally active, toxin portion of the CrylFa protein, and wherein a Cry1Da core toxin is the N-terminal, insecticidally active, toxin portion of the CrylDa protein.

7. Use of a composition of claim 6 comprising a host transformed to express both a CrylFa core toxin-containing protein and a CrylDa core toxin containing protein, wherein said host is a microorganism or a plant cell, wherein a CrylFa core toxin is the N-terminal, insecticidally active, toxin portion of the Cry1Fa protein, and wherein a CrylDa core toxin is the N-terminal, insecticidally active, toxin portion of the CrylDa protein.

8. A method of controlling lepidopteran pests comprising presenting to said pests or to the environment of said pests an effective amount of a composition of claim 6.

9. A transgenic plant that produces three insecticidal proteins, comprising a CrylDa insecticidal protein and a Cry1Fa insecticidal protein and a third insecticidal Cry protein, that are insecticidal to the same target insect, said insect having potential to develop resistance to any one of said Cry proteins, and wherein each said Cry protein binds a different gut receptor in said target insect.

10. The plant of claim 9 wherein said insect is fall armyworm.

11. A transgenic plant that produces a Cry1Fa protein plus a Cry1Da protein plus a third protein selected from the group consisting of Vip3A, Cry1C, CrylBe, and Cry1E proteins.

## Patentansprüche

1. Eine transgene Pflanze oder Pflanzenzelle, umfassend DNA, die ein CrylDa insektizides Protein kodiert und exprimiert, und DNA, die ein CrylFa insektizides Protein kodiert und exprimiert.

2. Ein Samen einer Pflanze des Anspruchs 1, wobei der Samen DNA, die ein CrylDa insektizides Protein kodiert und exprimiert, und DNA, die ein CrylFa insektizides Protein kodiert und exprimiert, umfasst.

3. Eine Mischung von Samen, umfassend Refugien-Samen von nicht-Bt-Refugien-Pflanzen und eine Vielzahl von Samen des Anspruchs 2 umfassend DNA, die ein CrylDa insektizides Protein kodiert und exprimiert, und DNA, die ein CrylFa insektizides Protein kodiert und exprimiert, wobei die Refugien-Samen weniger als 40 % aller Samen in der Mischung umfassen.

4. Ein Verfahren zum Handhaben der Entwicklung von Resistenz gegen ein Cry Toxin durch ein Insekt, das Verfahren umfassend das Pflanzen von Samen umfassend DNA, die ein CrylDa insektizides Protein kodiert und exprimiert, und DNA, die ein CrylFa insektizides Protein kodiert und exprimiert, um ein Pflanzenfeld zu produzieren.

5. Die transgene Pflanze des Anspruchs 1, die Pflanze weiterhin umfassend DNA, die ein Cry1Ab Kerntoxin-enthaltendes Protein kodiert, wobei ein Cry1Ab Kerntoxin der N-terminale, insektizid-aktive Toxin-Teil des CrylAb Proteins ist.

6. Verwendung einer Zusammensetzung zum Kontrollieren von Lepidoptera-Schädlingen, umfassend Zellen, die wirksame Mengen eines CrylFa Kerntoxin-enthaltenden Proteins und eines CrylDa Kerntoxin-enthaltenden Proteins exprimieren, wobei ein CrylFa Kerntoxin der N-terminale, insektizid-aktive Toxin-Teil des CrylFa Proteins ist, und wobei ein CrylDa Kerntoxin der N-terminale, insektizid-aktive Toxin-Teil des CrylDa Proteins ist.

7. Verwendung der Zusammensetzung nach Anspruch 6, umfassend einen Wirt, der transformiert ist, um ein CrylFa Kerntoxin-enthaltendes Protein und ein CrylDa Kerntoxin-enthaltendes Protein zu exprimieren, wobei der Wirt ein Mikroorganismus oder eine Pflanzenzelle ist, wobei ein CrylFa Kerntoxin der N-terminale, insektizid-aktive Toxin-Teil des CrylFa Proteins ist, und wobei ein CrylDa Kerntoxin der N-terminale, insektizid-aktive Toxin-Teil des CrylDa Proteins ist.

8. Verwendung eines Verfahrens zum Kontrollieren von Lepidoptera-Schädlingen, welches den Schädlingen oder der Umgebung der Schädlinge gegenüber ein Präsentieren einer wirksamen Menge einer Zusammensetzung nach Anspruch 6 umfasst.

9. Eine transgene Pflanze, die drei insektizide Proteine produziert, umfassend ein CrylDa Kerntoxin-enthaltendes Protein, ein CrylFa Kerntoxin-enthaltendes Protein und ein drittes insektizides Cry Protein, die insektizid für das gleiche Zielinsekt sind, wobei das Insekt das Potenzial hat, eine Resistenz gegen eines der Cry Proteine zu entwickeln, und wobei jedes der Cry Proteine einen unterschiedlichen Darmrezeptor in dem Zielinsekt bindet.

10. Die Pflanze des Anspruchs 9, wobei das Insekt der Herbst-Heerwurm ist.

11. Eine transgene Pflanze, die ein Cry1Fa, ein CrylDa und ein drittes Protein, ausgewählt aus der Gruppe bestehend aus den Vip3A, CrylC, CrylBe und Cry1E Proteinen, produziert.

## Revendications

1. Végétal ou cellule végétale transgénique comprenant un ADN codant et exprimant une protéine insecticide CrylDa et un ADN codant et exprimant une protéine insecticide CrylFa.

2. Semence d'un végétal conforme à la revendication 1, laquelle semence comprend un ADN codant et exprimant une protéine insecticide CrylDa et un ADN codant et exprimant une protéine insecticide CrylFa.

3. Mélange de semences comprenant des semences de refuge issues de végétaux de refuge non-Bt et un certain nombre de semences conformes à la revendication 2, comprenant un ADN codant et exprimant une protéine insecticide CrylDa et un ADN codant et exprimant une protéine insecticide CrylFa, dans lequel mélange lesdites semences de refuge représentent moins de 40 % de toutes les semences contenues dans le mélange.

4. Procédé visant à maîtriser le développement de la résistance à une toxine Cry chez un insecte, lequel procédé comporte le fait de mettre en terre des semences comprenant un ADN codant et exprimant une protéine insecticide CrylDa et un ADN codant et exprimant une protéine insecticide CrylFa, en vue de produire un champ de végétaux.

5. Végétal transgénique conforme à la revendication 1, lequel végétal comprend en outre un ADN codant une protéine qui comporte en son coeur une toxine de CrylAb, laquelle toxine coeur de CrylAb est la partie toxine N-terminale, dotée de l'activité insecticide, de la protéine CrylAb.

6. Utilisation, pour lutter contre des lépidoptères nuisibles, d'une composition comprenant des cellules qui expriment, en quantités efficaces, à la fois une protéine comportant en son coeur une toxine de CrylFa et une protéine comportant en son coeur une toxine de CrylDa, étant entendu que la toxine coeur de CrylFa est la partie toxine N-terminale, dotée de l'activité insecticide, de la protéine CrylFa et la toxine coeur de CrylDa est la partie toxine N-terminale, dotée de l'activité insecticide, de la protéine CrylDa.

7. Utilisation d'une composition, définie dans la revendication 6, qui comprend un hôte transformé pour pouvoir exprimer à la fois une protéine comportant en son coeur une toxine de CrylFa et une protéine comportant en son coeur une toxine de CrylDa, étant entendu que ledit hôte est un microorganisme ou une cellule végétale, et que la toxine coeur de CrylFa est la partie toxine N-terminale, dotée de l'activité insecticide, de la protéine CrylFa et la toxine coeur de CrylDa est la partie toxine N-terminale, dotée de l'activité insecticide, de la protéine CrylDa.

8. Procédé de lutte contre des lépidoptères nuisibles, comprenant le fait de mettre lesdits nuisibles, ou l'environnement de ces nuisibles, en présence d'une composition définie dans la revendication 7, utilisée en une quantité efficace.

9. Végétal transgénique qui produit trois protéines insecticides, comprenant une protéine insecticide CrylDa, une protéine insecticide CrylFa et une troisième protéine Cry insecticide, qui sont insecticides pour le même insecte cible, étant entendu que ledit insecte peut développer une résistance à n'importe laquelle de ces protéines Cry et que chacune de ces protéines Cry se lie à un récepteur intestinal différent chez ledit insecte cible.

10. Végétal conforme à la revendication 9, pour lequel ledit insecte cible est la légionnaire d'automne.

11. Végétal transgénique qui produit une protéine CrylFa et une protéine CrylDa, avec en plus une troisième protéine choisie dans l'ensemble formé par les protéines Vip3A, CrylC, Cry1Be et Cry1E.
